# EUROPEAN PATENT APPLICATION

(11) **EP 2 075 016 A1**
(43) Date of publication of application: **01.07.2009**
(21) Application number: 07022013.2
(22) Date of filing: 13.11.2007
(51) Int. Cl.: A61L 31/06

(54) **Branched polymers in medical devices**

(71) Applicant: Abbott Laboratories Vascular Enterprises Limited, Terrace, Dublin 2 (IE)
(72) Inventor: Lorentz, Günter Dr., 72072 Tübingen (DE)
(74) Representative: Peters, Hajo

(57) **Abstract**

The present invention refers to medical devices comprising non-linear block-copolymers especially those selected form branched polyamides, branched or grafted Block-Co-Polymers as well as dendritic systems carrying polyamides, wherein the materials are having a high flexibility and a high stress resistance, especially tensile strength or tear resistance, allowing their use in medical devices, especially in balloons attached to a balloon catheter.

## Description

### Field of the invention

The present invention refers to medical devices comprising nonlinear block-co-polymers like branched polyamides, branched or grafted Block-Co-Polymers as well as dendritic systems carrying polyamides, wherein the materials are having a high flexibility and a high stress resistance, especially tensile strength or tear resistance, allowing their use in medical devices, especially in catheters or in balloons attached to a balloon catheter for angioplastic applications.

### Background of the invention

Polyamides or polyamide elastomers have been used in the polymer industry for a long time and - due to their enormous range of possible applications - are found in many branches of industrial products. Recently in the area of medicinal devices good use has been made of these materials especially in devices/implants like the balloons on a balloon catheter. The most popular polyamides used include different sorts of Nylons or Coploymers such as PEBAX®. Even though these materials have certainly been used successfully, due to the strains put on the materials and the necessity to improve their characteristics in the light of growing experience coming from increasing numbers of treated patients, there clearly is a need for improved materials allowing for an effective treatment of the patient minimizing risks, preferably with an economical production process.

With the focus of this invention on balloon material for balloon catheters one of the main parameters of a balloon is compliance, the change of the balloon diameter with rising inflation pressure; as used herein three categories are being identified:
➢ Non-compliant (NC) with a diameter increase of up to 0.55% per bar;
➢ Semi-compliant (SC) with a diameter increase of between 0.55 to 0.8 % per bar;
➢ Compliant with a diameter increase over 0.8 % per bar
as the balloon is pressurized from an inflation pressure between the nominal pressure and rated burst pressure.

While a certain level of compliance is needed to allow the compression of the arterio-sclerotic plaque in a vessel, an amount of pressure expressed on the stenosis as executed by a more non-compliant balloon is also needed. As also semi-compliant and compliant balloons are more prone to failure during PTCA and also "dog-boning", an inflation of the balloon outside the stenotic area of the vessel resulting sometimes in devastating stress on the healthy part of the vessel, a more non-compliant parameter is wanted.

### Summary of the invention

It is an object of the current invention to provide new polymers or to identify polymers having high flexibility and high stress resistance, especially tensile strength or tear resistance in addition to the good physical characteristics of the known polyamide elastomers. As the special focus of this invention is on the search for new materials in balloons for balloon catheters used in PTCA (Percutaneous transluminal coronary angioplasty) a material with suitable compliance needs to be identified.

The invention thus refers to a medical device comprising a non-linear block-copolymer, especially to a balloon attached to a balloon catheter like those used in PTCA/angioplastic applications.

The invention further resides in a non-linear block-co-polymer being selected from a branched polyamide, a branched or grafted Block-Co-Polymers or a dendritic system carrying polyamides, wherein the branched polyamide, the branched or grafted Block-Co-Polymer or the dendritic system carrying polyamides comprise at least two hard segments and at least one functionalized soft segment.

The invention furthermore resides in the use of a polymer according to the invention in the production of medical devices, balloon material, stents, stent grafts, and catheters.

### Detailed Description of the invention

The use of stents, balloons, catheters and other medical devices etc. in minimal invasive surgery, especially in the cardiovascular field, has in the last years shown a high growth. As a consequence the need for useful materials fulfilling highly specialized needs in the field of different medicinal devices has clearly risen in a technical area, which traditionally is more governed by bulk products. Especially in the field of cardiovascularily used balloons there was a clear desire for an elastomer, which is on one hand flexible enough to be introduced into a vascular environment without causing damage, while on the other hand being stable and rigid enough, especially in the moment of actual surgery, inflation in the vessel, to not be extended too much inside the vessel. Especially a suitable compliance, the change of the balloon diameter with rising inflation pressure, especially with a flat rise in the compliance curve (pressure/diameter) is needed.

There are 3 kinds of material used nowadays for medical devices, especially balloons, over which the material of the current invention - if compared case by case - shows advantages.
a) Nylon: Over Nylon, coming in different sorts, especially Nylon-12, the polymers of the invention show the advantage, that they are more flexible and/or have a lower water absorption. Especially the lack of flexibility is often considered as a drawback in medical devices using Nylon.
b) PEBA: Over PEBA (e.g. PEBAX^{®}) the polymers of the invention show the advantage, that they are slightly more rigid and/or have a lower water absorption, again making them superior for the intended special use and allowing a much needed compromise balancing flexibility and rigidity. In addition the material of the invention seem to show higher stability, especially if compared to the effects of thermo-oxidation shown by PEBA and/or also an improved dimensional stability. Also, producing a compound according to the invention needs one polymerization step less than known from PEBA, resulting in the possibility of lower production costs.
c) Blend of a) and b): The need for a compromise between the higher rigidity of Nylon and higher flexibility of PEBA has already resulted in blends being used. A disadvantage of blends is that the phases tend to show phase separation that leads to unstable morphology, whereas on the other hand the material used according to the invention leads to a stabilized morphology.

Especially this need for a compromise between the higher rigidity of Nylon and higher flexibility of PEBA is at the focus of this invention, and thus, to find or identify materials showing - especially as balloon material - features - like the E-Modulus - situated between those of Nylon and PEBA and suitable as balloon material. Especially in regards to compliance a more non-compliant behaviour is needed lying closer to Nylon than to PEBA, this also being true for longitudinal growth.

The invention thus refers to a medical device comprising a non-linear block-copolymer. Thereby it is preferred if the medical device according to the invention is selected from implanted or implantable medical devices, preferably balloon/balloon material, stents, stent grafts, grafts, graft connectors or catheters, most preferably is a balloon/balloon material.

As shown below non-linear block-co-polymers are surprisingly showing the features as needed to be suitable as balloon material and physical features situated between those of Nylon and PEBA. Especially a) the E-moduli are lying between those of Nylon and those of PEBA clearly indicating that balloons made of the material according to the invention are not as rigid as those made of Nylon nor as flexible as those made of PEBA , b) the compliance curve of balloons made of the material according to the invention is showing only a slow rise indicating a low compliance and c) the longitudinal growth is low lying closer to Nylon.

In the context of this invention "non-linear block-co-polymer" is defined as a polymer being build from at least two different (and distinct) blocks of polymers (from hereon also called "segments"), wherein one block is either directly or through a coupling reagent covalently bound to at least three distinct blocks (segments) of polymers.

In the context of this invention "segment" is defined as a separated/ distinct block of polymer. Accordingly "hard segment" is a segment with relatively high shore hardness, e.g. like a polyamide, and "soft segment" is a segment with relatively low shore hardness, e.g. like a polyether, a polyester; a polydimethylsiloxane or a siloxylated polyether diole.

In the context of this invention "coupling reagent" is defined as a compound allowing by having at least two "functional" groups the coupling (covalent binding) of one block of polymer (segment) to at least one other block (segment) of polymers. In this regard they a "coupling reagent usually binds to a "functional" group of a "functionalized" block/segment. Examples of coupling reagents include biphenyl tetracarboxylic dianhydride; tris(2-aminoethyl)amine; trimethylpropane trisaminopropylene glycol ether; glycerol-propoxylate-triglycidylether; carbonyl biscaprolactam; or 1,3-phenylbisoxazolin; 1,4-phenylbisoxazolin. Included in the group of coupling reagents are "linkers".

In the context of this invention "linker" is defined as a "coupling reagent" having at least three "functional" groups and thus allowing the coupling (covalent binding) of one block of polymer (segment) to at least two other distinct blocks (segments) of polymers. Examples of linkers include biphenyl tris(2-aminoethyl)amine; trimethylpropane trisaminopropylene glycol ether; or glycerol-propoxylate-triglycidylether.

In the context of this invention "functional group" is defined as a chemical substituent bound to a block/segment of a polymer allowing the coupling (covalent binding) of this block/segment of polymer to another block (segment) of polymer or to a coupling reagent/linker. Examples of functional groups include epoxides, OH, COOH, NH₂, or others.

Accordingly "functionalized" in connection to a segment, especially a soft or hard (e.g. a polyamide) segment, means that the segment is carrying either by itself or after treatment with a functionalizing reagent at least one functional group.

According to the invention "functionalizing reagent" is defined as a reagent transferring to a segment at least one functional group. Preferably the functionalizing reagent is itself showing at least two functional groups. Examples of functionalizing reagents include aliphatic diamines (e.g. octadecyldiamine); 2-piperazinoethylamine; or trimellitic anhydride.

In a highly preferred embodiment of the medical device according to the invention the medical device is a balloon attached to a balloon catheter, like a catheter for angioplastic applications.

In another preferred embodiment of the medical device according to the invention the balloon is consisting of the non-linear block-co-polymer.

In another preferred embodiment of the medical device according to the invention the non-linear block-co-polymer is selected from branched polyamides, a branched or grafted Block-Co-Polymer or a dendritic system carrying polyamides.

Another aspect of the current invention provides a non-linear block-co-polymer according to the invention being selected from a branched polyamide, a branched or grafted block-co-polymer or a dendritic system carrying polyamides.

In a preferred embodiment of the non-linear block-co-polymer according to the invention the block-co-polymer comprises at least three hard segments covalently bound directly or through a linker to at least one soft segment;
or
at least three soft segments covalently bound directly or through a linker to at least one hard segment.

In another preferred embodiment of the non-linear block-co-polymer according to the invention the non-linear block-co-polymer has a structure selected from one of the following general formulas: Type II A or B, Type III A or B, Type IV A or B, Type V A or B, Type VI A or B, Type VII A or B, Type VIII A or B; Type IX A or B, or Type X: 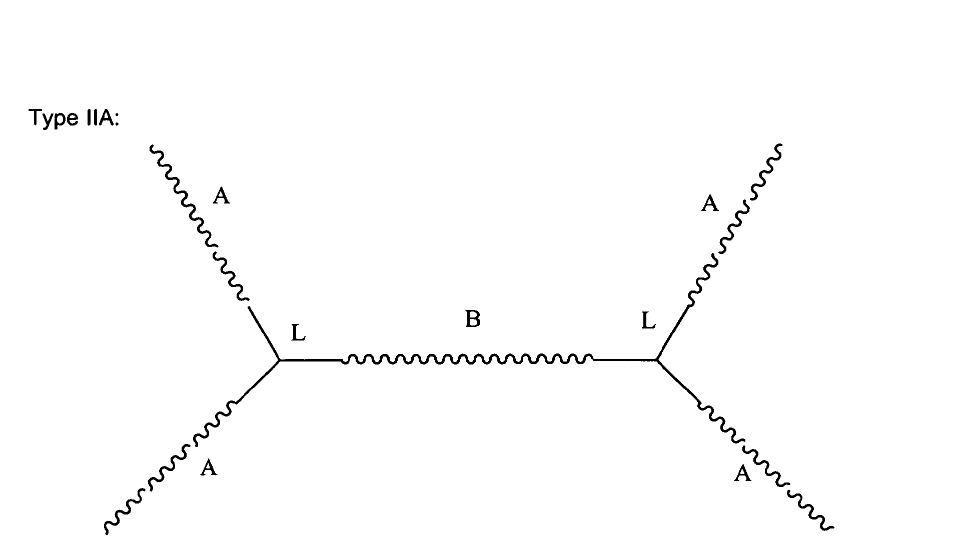 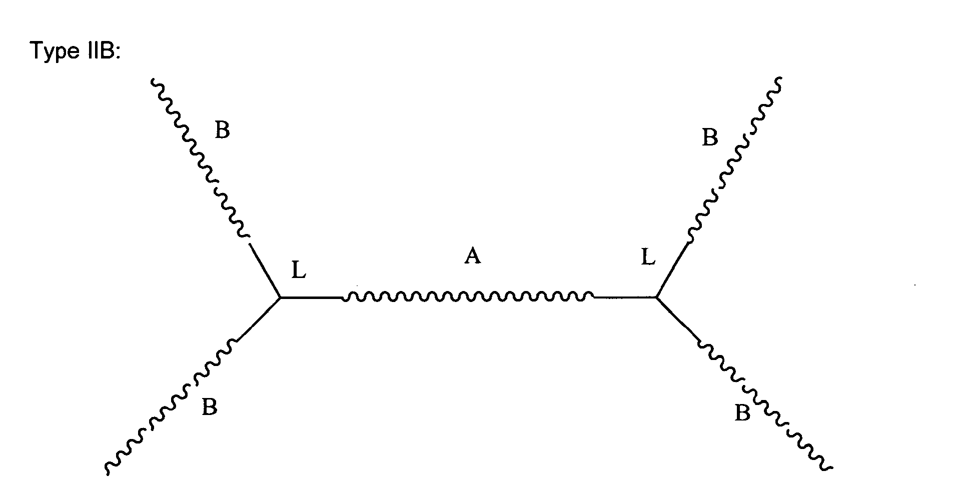 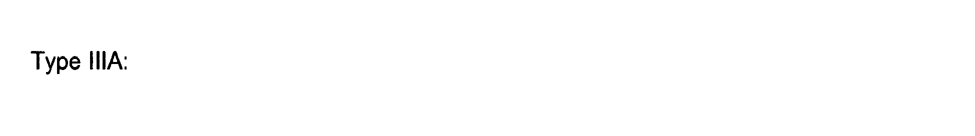 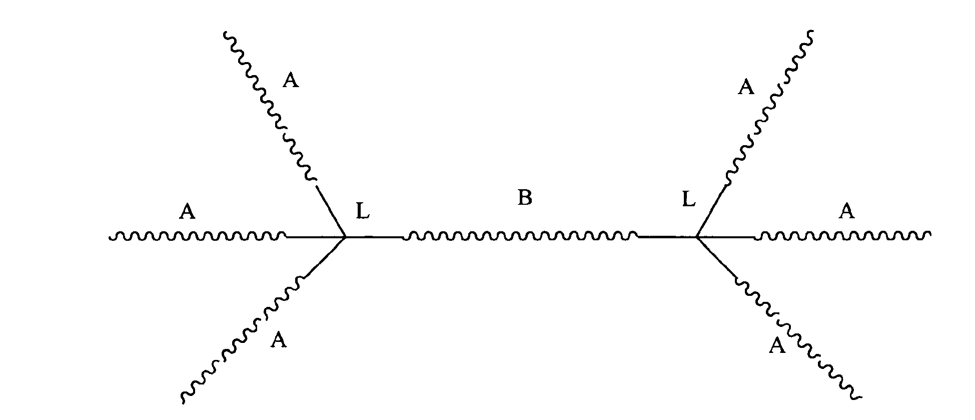 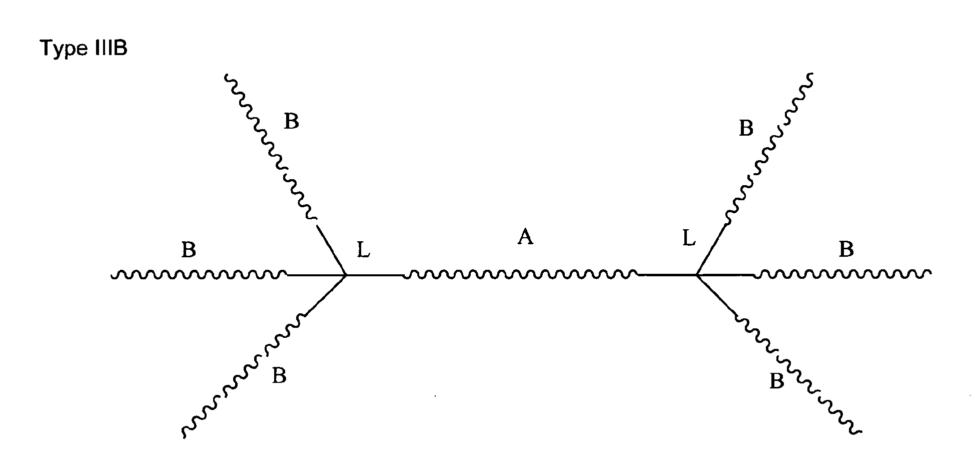 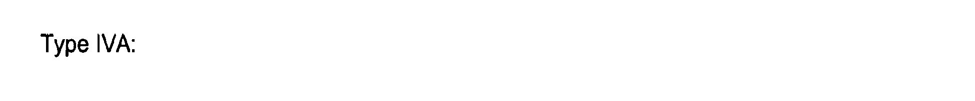 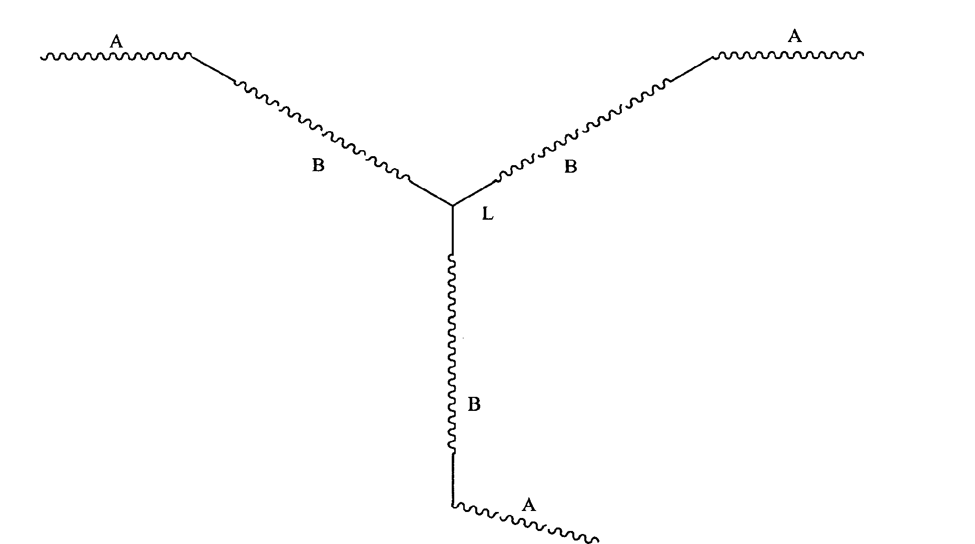 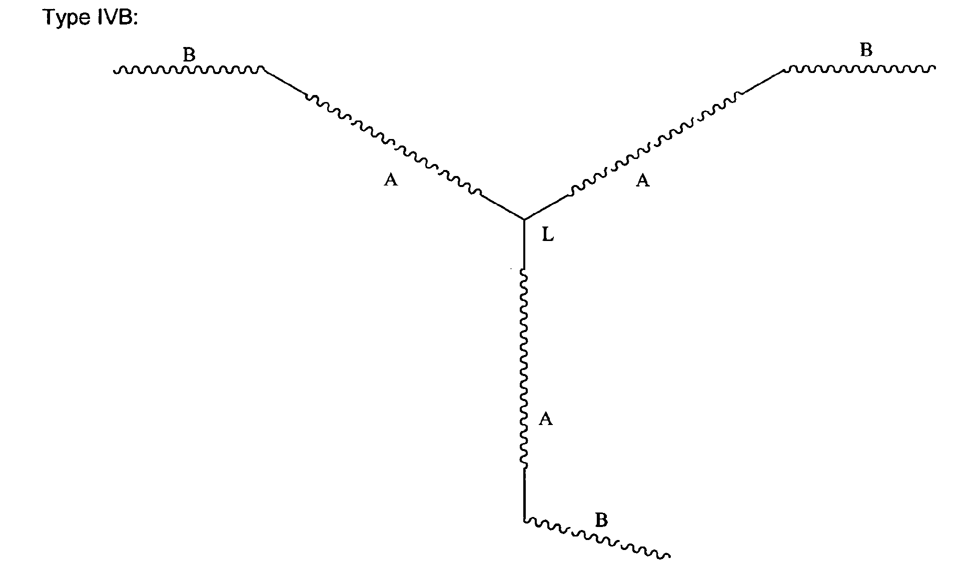 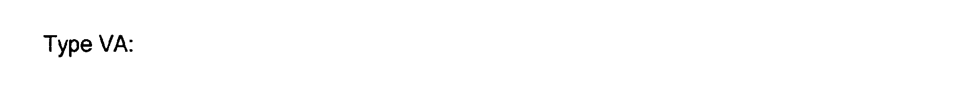 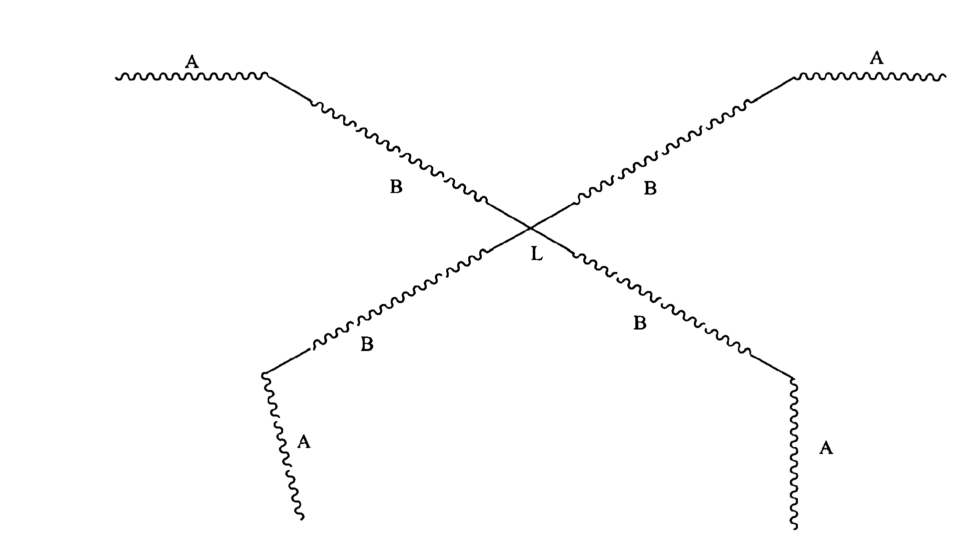 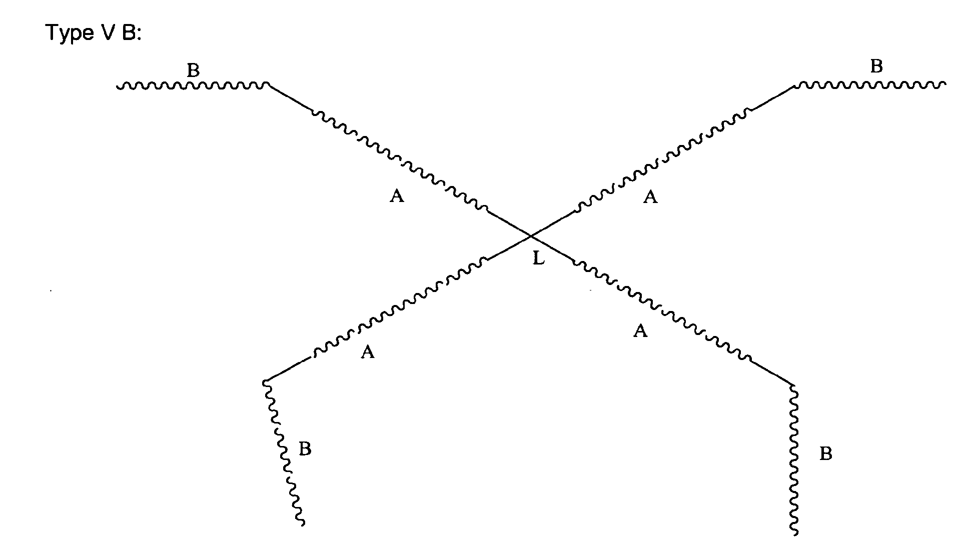 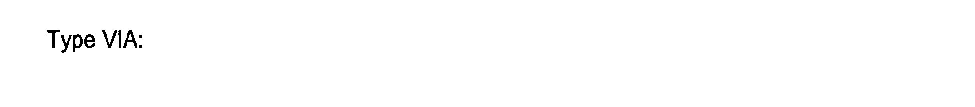 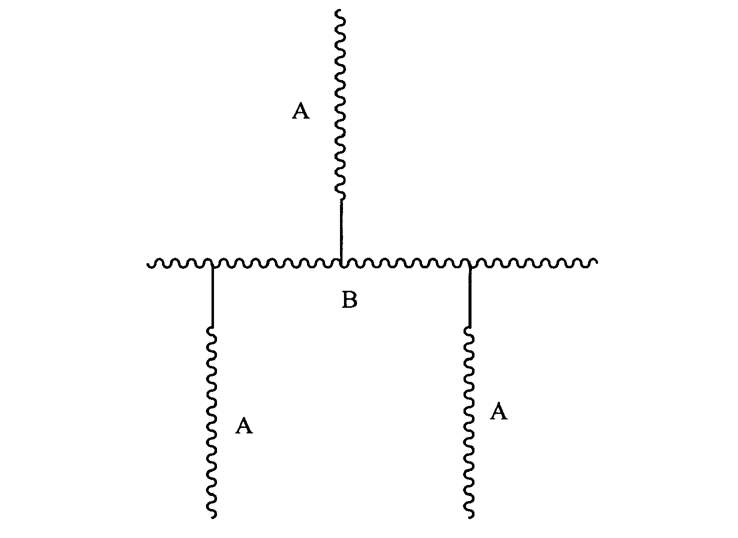 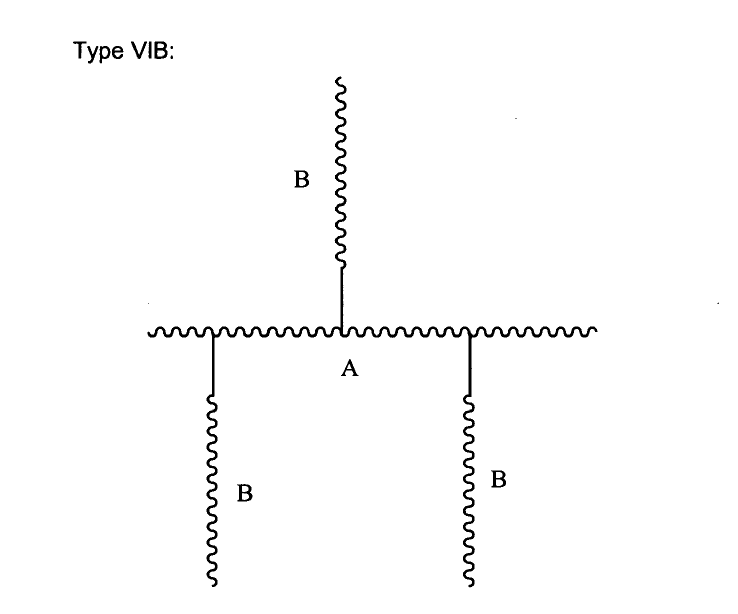 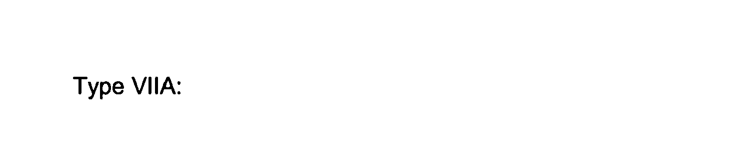 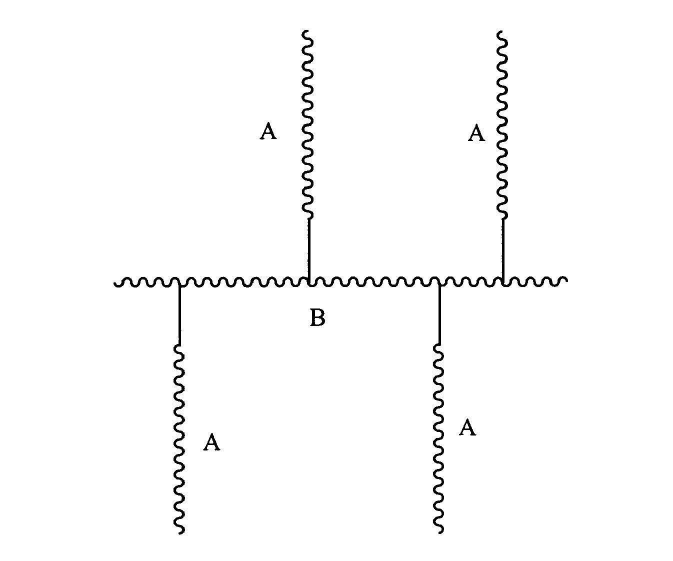 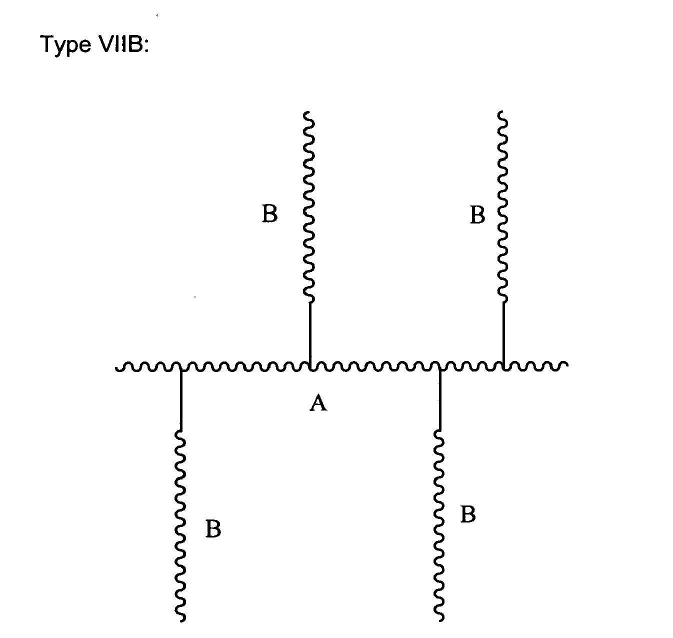 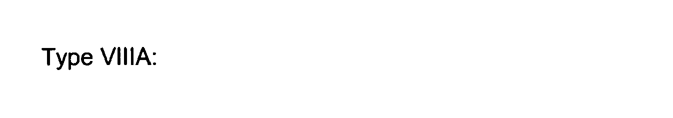 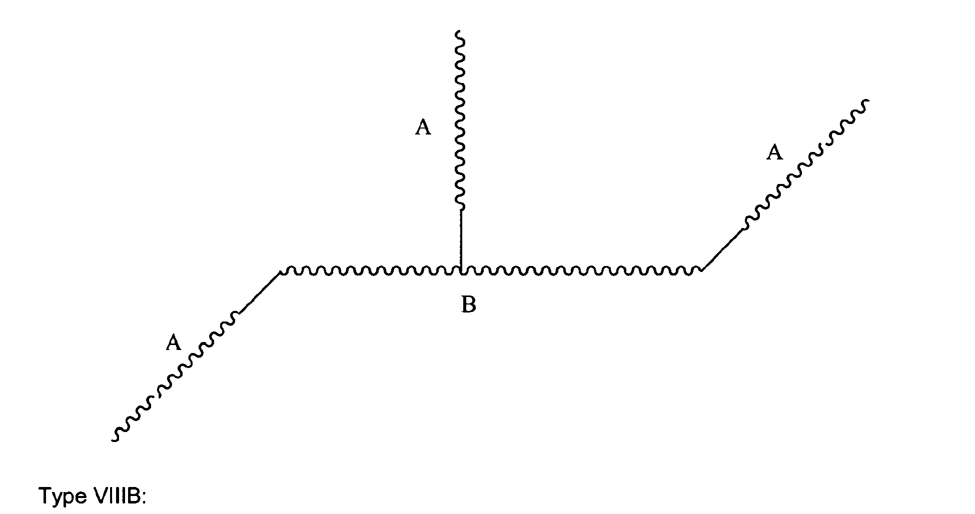 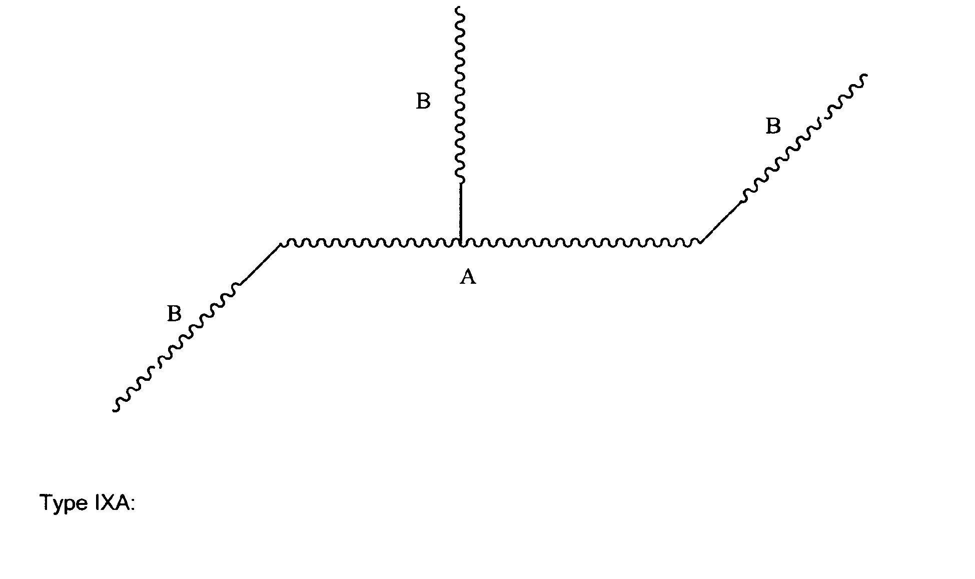 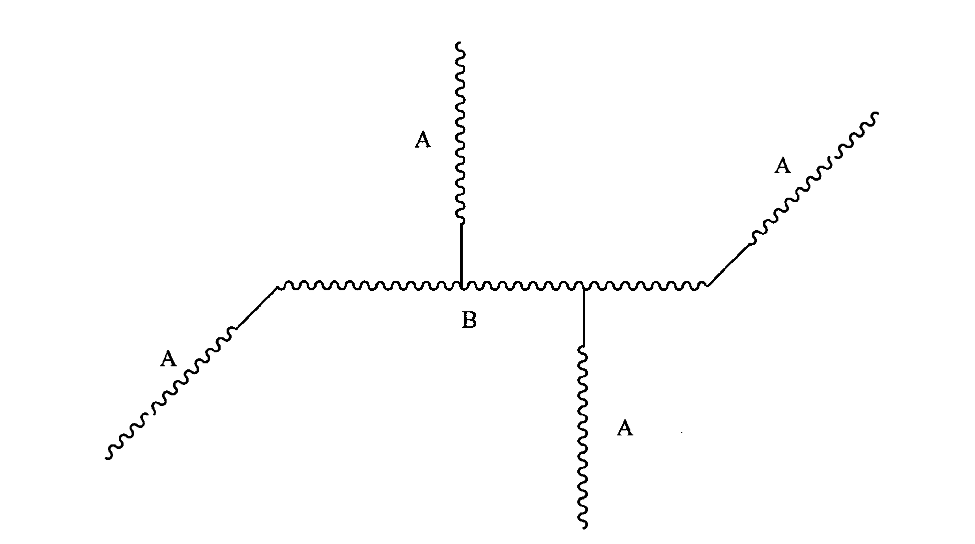 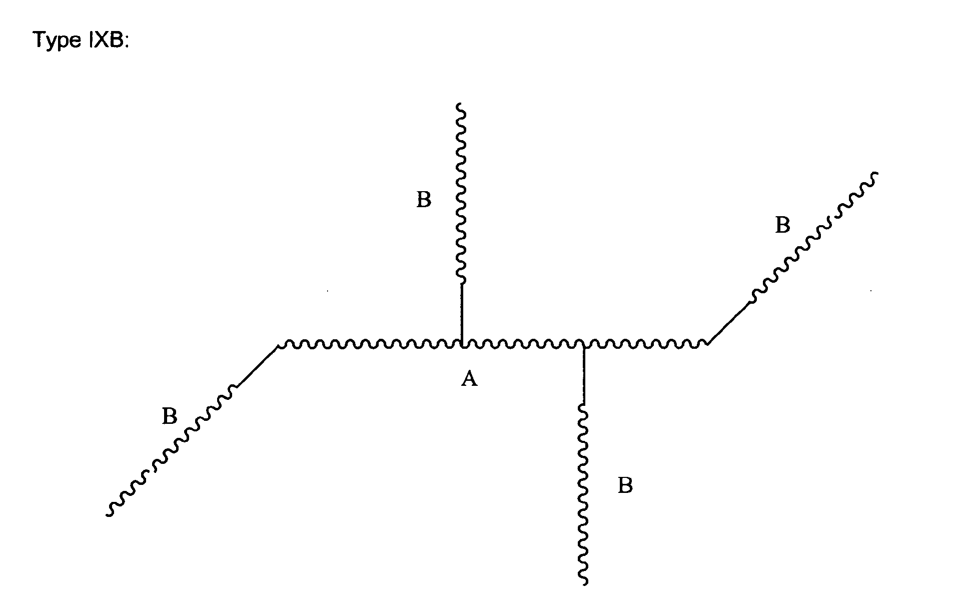 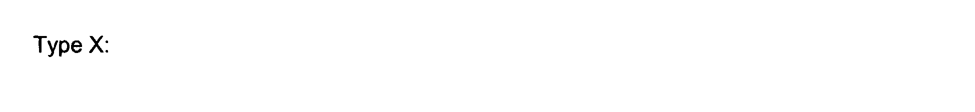 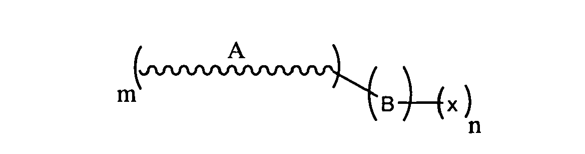 , wherein
A is a hard segment,
B is a soft segment;
x is a functional group;
m is a number between 3 and 15,
n is a number between 0 and 60;
and n+ m is a number between 3 and 70;
_ is an optional coupling reagent; and
L is a linker.

In a preferred embodiment the Type X is covering dendritic-derivatives in which the (B) is a soft segement formed by a dendritic polymer. The hard segments are connected to at least three or more (but not necessary all) of the functional (end) groups of the dendritic molecule. Dendritic molecues are star-shaped molecules branched regularly and in form of a cascade with a radial symmetry. In principle a dendritic molecule has just one core from which at least 3 branches (dendrons) branch-off. Dendrons are formed by one branch of further branched sub-units, which are connected through one line to the core. For an in-depth definition on dendritic molecules it is referred to H.G. Elias, Makromeleküle, Page 50-52, Band 1, 6. Aufl., 1999, Wiley-VCH.

One theoretical example of a dendritic molecule is depicted below:

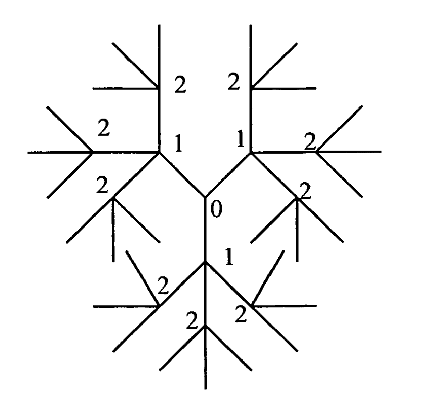

The end of the bonds may be further branched and finally end in a functional group.

One possible example of a dendritic molecule is Boltorn H40 (see below) with OH as functional group. Thus, compounds falling under Type X with Boltorn H40 would have the Boltorn H40 as soft segment with x (functional group) being OH and with Boltorn H40 being connected to at least 3 hard segments and with n + m being (theoretically) 64.

In another preferred embodiment of the non-linear block-co-polymer according to the invention the non-linear block-co-polymer comprises at least three hard segments covalently bound directly or through a coupling reagent to at least one soft segment.

In another preferred embodiment of the non-linear block-co-polymer according to the invention the block co-polymer has a structure selected from one of the following general formulas: Type II A, Type III A, Type IV A, Type V A, Type VI A, Type VII A, Type VIII A, Type IX A and Type X: 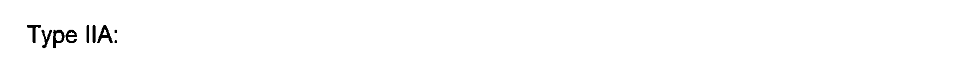 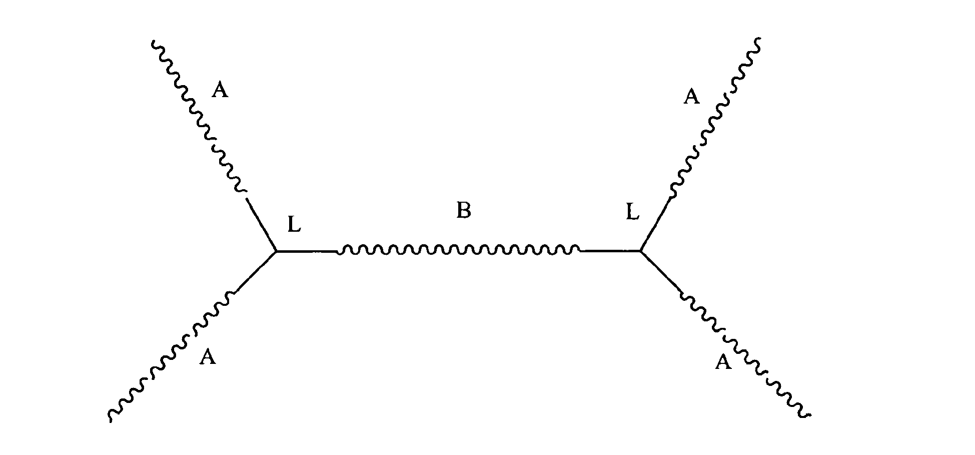 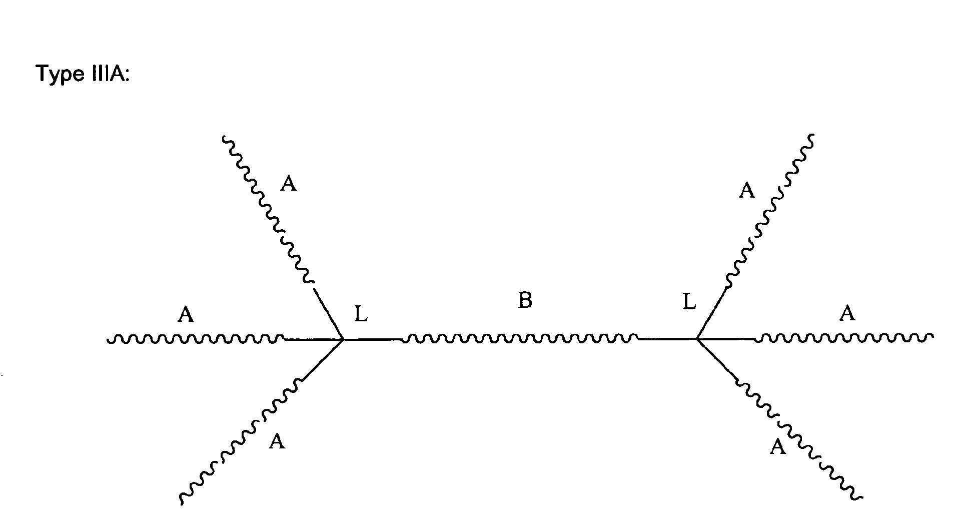 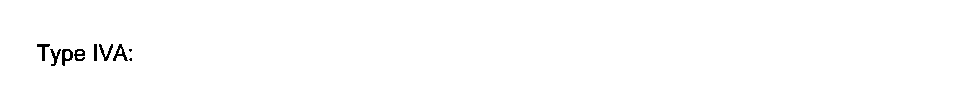 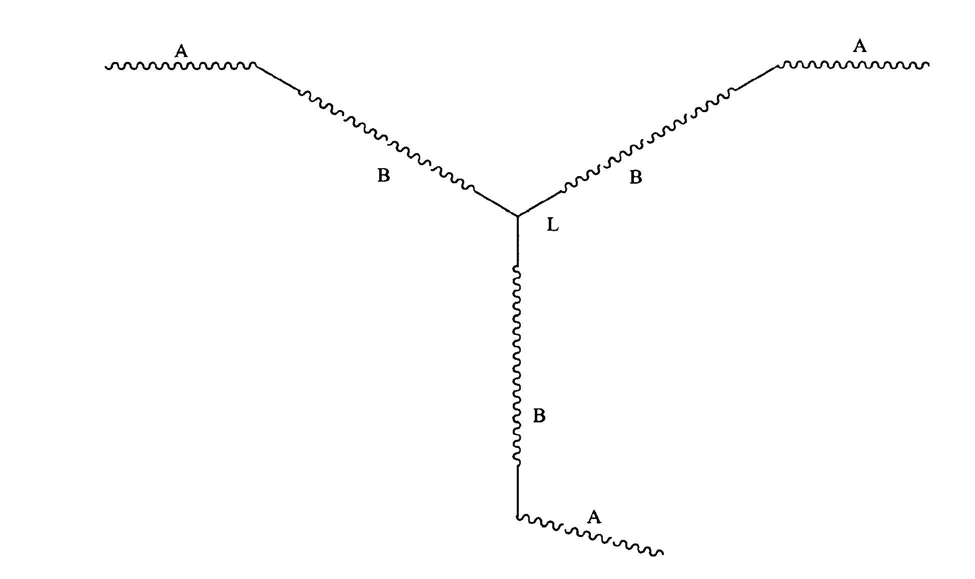 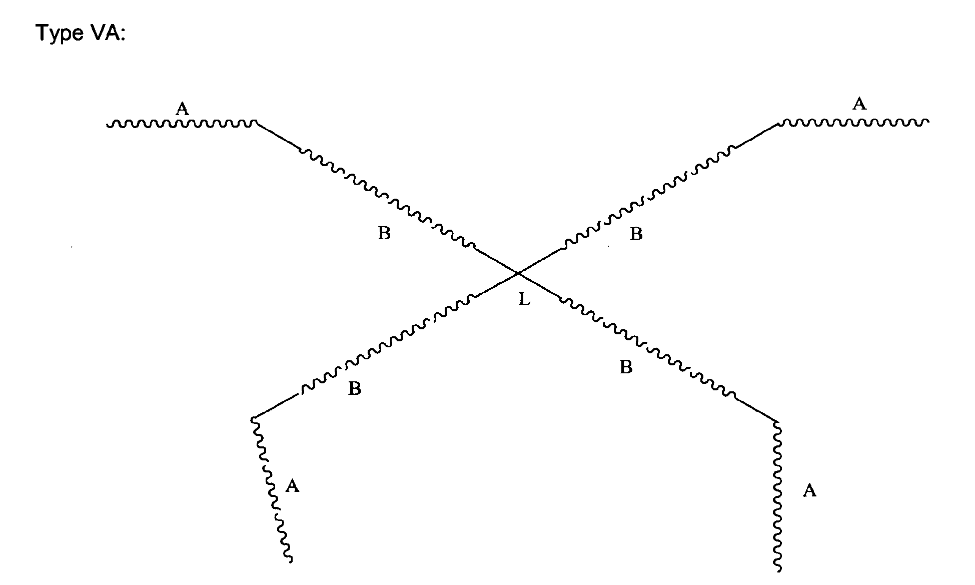 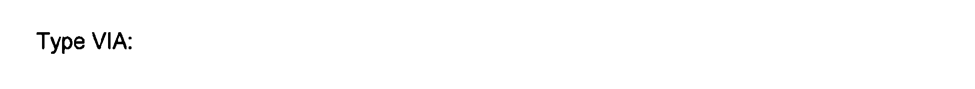 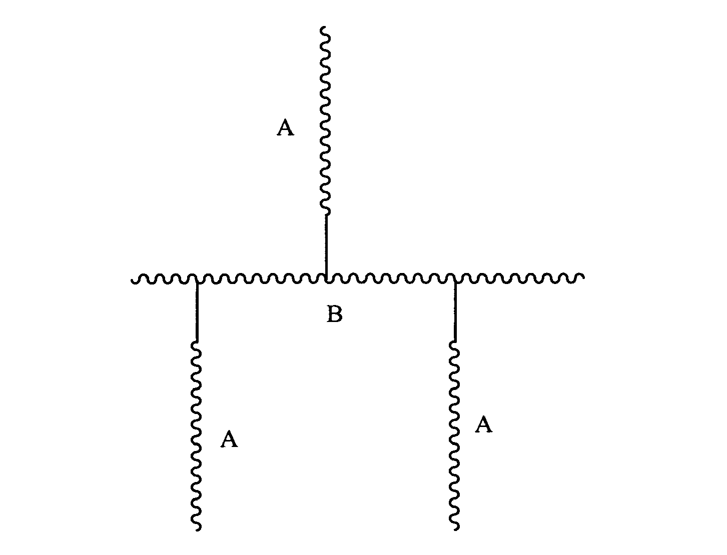 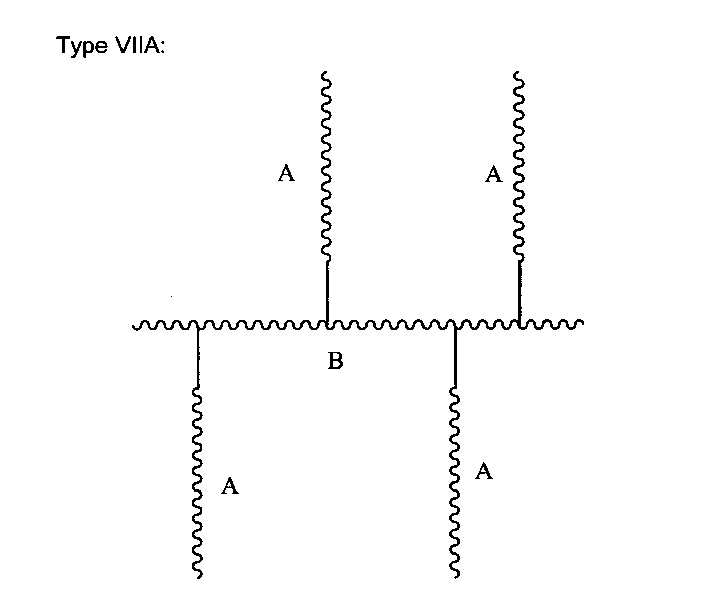 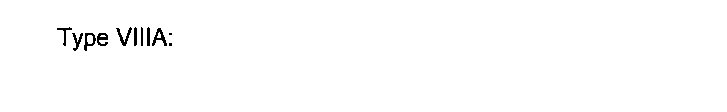 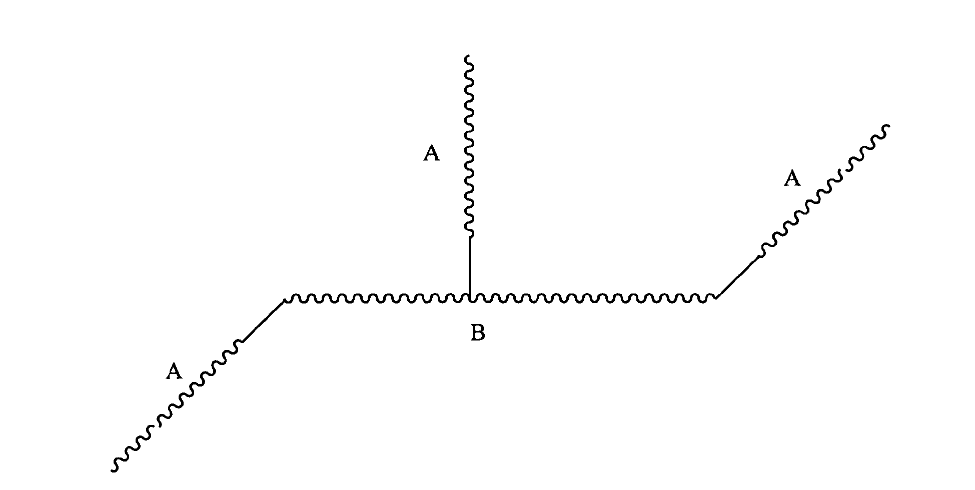 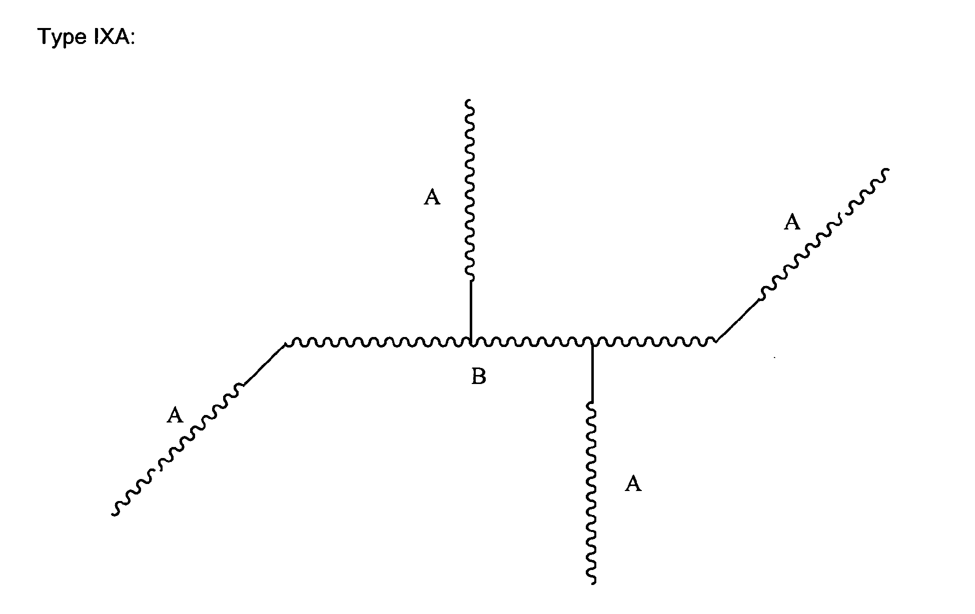 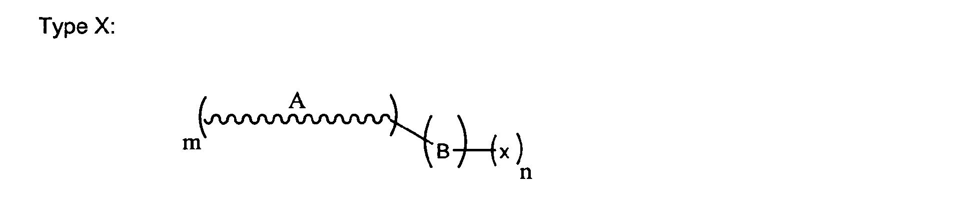 , wherein
A is a hard segment,
B is a soft segment;
x is a functional group;
m is a number between 3 and 15,
n is a number between 0 and 60;
and n+ m is a number between 3 and 70;
_ is an optional coupling reagent; and
L is a linker.

In another preferred embodiment of the non-linear block-co-polymer according to the invention in the non-linear block-co-polymer the hard segments are functionalized; preferably are functionalized showing at least one reactive group selected from epoxide, COOH, NH₂, or OH; more preferably are mono-functionalized, most preferably are mono-functionalized showing at least one reactive group selected from epoxide, COOH, NH₂, or OH.

In another preferred embodiment of the non-linear block-co-polymer according to the invention in the non-linear block-co-polymer the hard segments are polyamides, preferably are functionalized polyamides; preferably are functionalized polyamides showing at least one reactive group selected from epoxide, COOH, NH₂, or OH; most preferably are mono-functionalized polyamides showing at least one reactive group selected from epoxide, COOH, NH₂, or OH.

In another related preferred embodiment of the non-linear block-co-polymer according to the invention the polyamides are functionalized by a reagent selected from
● aliphatic diamines like octadecyldiamine;
● 2-piperazinoethylamine; or
● trimellitic anhydride.

In another preferred embodiment of the non-linear block-co-polymer according to the invention in the non-linear block-co-polymer the functionalized polyamides are low-molecular polyamides. "Low molecular" polyamides are defined as polyamides with a molecular weight of 1 to 15 kDa, preferably of 2 to 10 kDa.

In another preferred embodiment of the non-linear block-co-polymer according to the invention in the non-linear block-co-polymer at least one of the hard segments is covalently bound through a coupling reagent to at least one soft segment, wherein the coupling reagent is preferably selected from
- biphenyl tetracarboxylic dianhydride;
- tris(2-aminoethyl)amine;
- trimethylpropane trisaminopropylene glycol ether;
- glycerol-propoxylate-triglycidylether;
- carbonyl biscaprolactam; or
- 1,3-phenylbisoxazolin; 1,4-phenylbisoxazolin.

In another preferred embodiment of the non-linear block-co-polymer according to the invention in the non-linear block-co-polymer the soft segment/s is/are functionalized soft segment/s; preferably is/are functionalized soft segments selected from
- Polyethers;
- Polyesters;
- Polydimethylsiloxanes; or
- Siloxylated polyether dioles
preferably selected from
- Polyether;
- Polyethylenoxid-Polypropyleneoxid-Copolymer;
- Polytetramethyleneoxyde (Polytetrahydrofurane);
- Polyester;
- Tetra-OH-functionalized polyester;
- Dendritic Polyester;
- Polycaprolactone;
- Polydimethylsiloxane; or
- Siloxylated polyether diole,
most preferably selected from
- Polyethylenoxid-Polypropyleneoxid-Copolymer; or
- Dendritic Polyester Boltorn H40.

In a highly preferred embodiment of the non-linear block-co-polymer according to the invention in the non-linear block-co-polymer the hard segments are polyamides functionalized by 1-Octadecylamine and preferably coupled to 1,3-Phenylbisoxazoline or 1,4-Phenylbisoxazoline.

In another highly preferred embodiment of the non-linear block-co-polymer according to the invention in the non-linear block-co-polymer the soft segment segement is selected from Polyethylenoxid-Polypropyleneoxid-Copolymer; or Boltorn H40 and the hard segments are a polyamide functionalized by 1-Octadecylamine and coupled to 1,3-Phenylbisoxazoline.

In a highly preferred embodiment of the medical device according to the invention the non-linear block-co-polymer is a non-linear block-co-polymer according to the invention as described above.

Another aspect of the current invention provides a block-co-polymer "Z", wherein the polymer is of general formula Type IA or Type IB 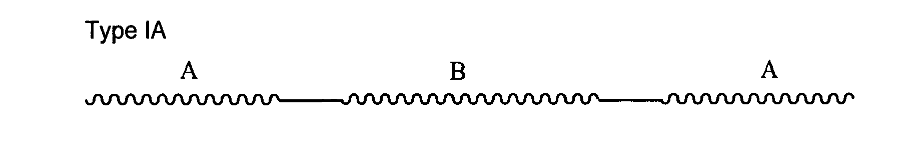 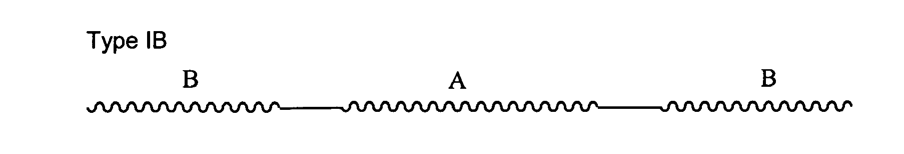 , wherein
A is a hard segment; preferably a functionalized polyamide;
_ is an optional coupling reagent; and
B is a soft segment, preferably a functionalized polyamide.

Another related aspect of the current invention provides a medical device comprising a block-co-polymer "Z". Here it is preferred if the medical device according this invention is selected from implanted or implantable medical devices, preferably balloon/balloon material, stents, stent grafts, grafts, graft connectors or catheters, more preferably is a balloon/balloon material, most preferably is a balloon attached to a balloon catheter.

Another aspect of the current invention provides the use of a non-linear block-copolymer, selected from branched polyamides, a branched or grafted Block-Co-Polymer or a dendritic system carrying polyamides in the production of implants or medical devices.

In a highly preferred embodiment of the use according to the invention the medical are preferably selected from balloons/balloon material, stents, stent grafts, grafts graft connectors , filters, embolic protection devices, closure devices, delivery systems, catheters and medical tubings, most preferably from balloons, balloon materials, catheters or medical tubings.

"Balloon or balloon material" in the context of this invention especially means a balloon like those used in coronary balloon angioplasty and the material used for these balloons, especially balloon catheters. In this, e.g. a balloon catheter is inserted into an artery or other lumen and advanced to e.g. a narrowing in a coronary artery. The balloon is then inflated to enlarge the lumen.

"Stent" means an elongate implant with a hollow interior and at least two orifices and usually a circular or elliptical, but also any other, cross section, preferably with a perforated, lattice-like structure that is implanted into vessels, in particular blood vessels, to restore and maintain the vessels patent and functional.

"Graft" means an elongate implant with a hollow interior and with at least two orifices and usually circular or elliptical, but also any other, a cross section and with at least one closed polymer surface which is homogeneous or, optionally, woven from various strands. The surface preferably is impermeable to corpuscular constituents of blood and/or for water, so that the implant serves as a vascular prosthesis and is usually employed for damaged vessels or in place of vessels.

"Stent graft" means a connection between a stent and a graft. A stent graft preferably comprises a vascular prosthesis reinforced with a stent (both as defined above), wherein a polymer layer is homogeneous or, optionally, woven, knitted plaited etc. from various strands and is either impermeable for corpuscular constituents of blood and/or for water or can also be permeable. More preferably, the stent has on at least 20% of its surface a perforated (lattice-like), preferably metallic, outer layer and at least one closed polymer layer that is located inside or outside the stent outer layer. The closed polymer layer may be homogeneous or, optionally, woven from various strands, and is impermeable for corpuscular constituents of blood and/or for water. Optionally, where the closed polymer layer is disposed inside the metallic outer layer, a further perforated (lattice-like), preferably metallic, inner layer may be located inside the polymer layer.

"Graft connector" means an implant that connects at least two hollow organs, vessels or grafts, consists of the materials defined for grafts or stent grafts and/or has the structure defined for the latter. Preferably, a graft connector has at least two, three or four, orifices, arranged, for example, as an asymmetric "T" shape.

"Catheter" means a tubular instrument intended for introduction into hollow organs. More preferably, a catheter may be designed for use in guiding other catheters, or for angiography, ultrasound imaging, or - especially - balloon catheters for dilatation or stent delivery. This includes also a "Catheter pump" meaning a catheter provided on its tip with a propeller able to assist the pumping of the myocardium.

In a highly preferred embodiment of the use according to the invention the non-linear block-co-polymer is a polymer according to the invention as described above or is a block-co-polymer "Z" according to the invention, as described above.

The examples and figures in the following section are merely illustrative and the invention cannot be considered in any way as being restricted to these applications.

### Figures:

- **Figure 1**: depicts the result of a test of the E-modulus in radial and longitudinal dimension of Examples 1A) and 2A) together with the comparators PEBAX and Nylon 12, Grillamide L25 (L25) with n=10.
- **Figure 2**: depicts the compliance curve of increase in balloon diameter against inflation pressure of Examples 1A) and 2A) together with the comparators PEBAX and Nylon 12 (PA12) with n=2.
- **Figure 3**: depicts the result of a test of longitudinal growth with pressure of Examples 1A) and 2A) together with the comparators PEBAX and Nylon 12, Grillamide L25 (L25) with n=8.

### Examples:

General Examples showing the general Formula Types I to IV and the reaction leading to them:

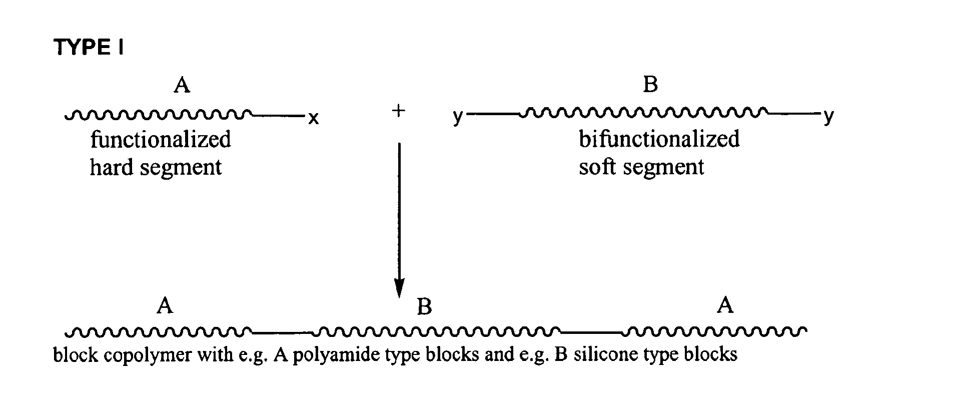

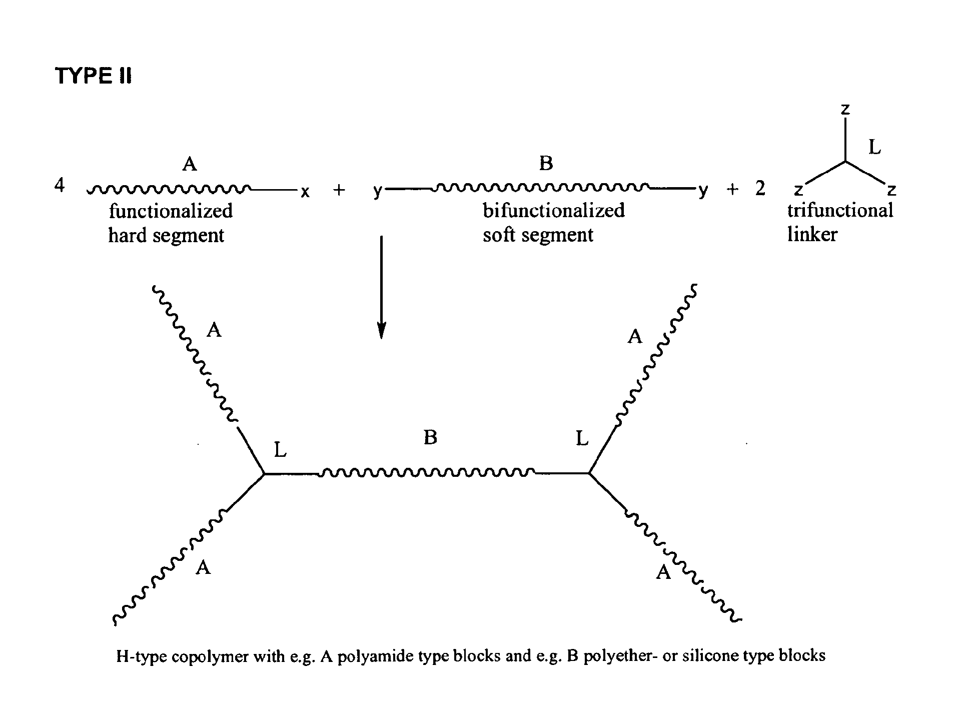

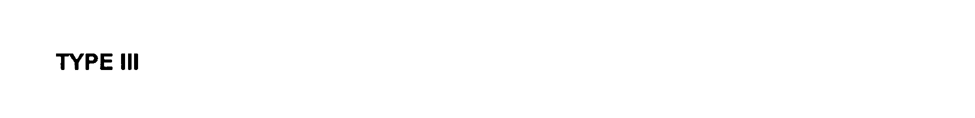

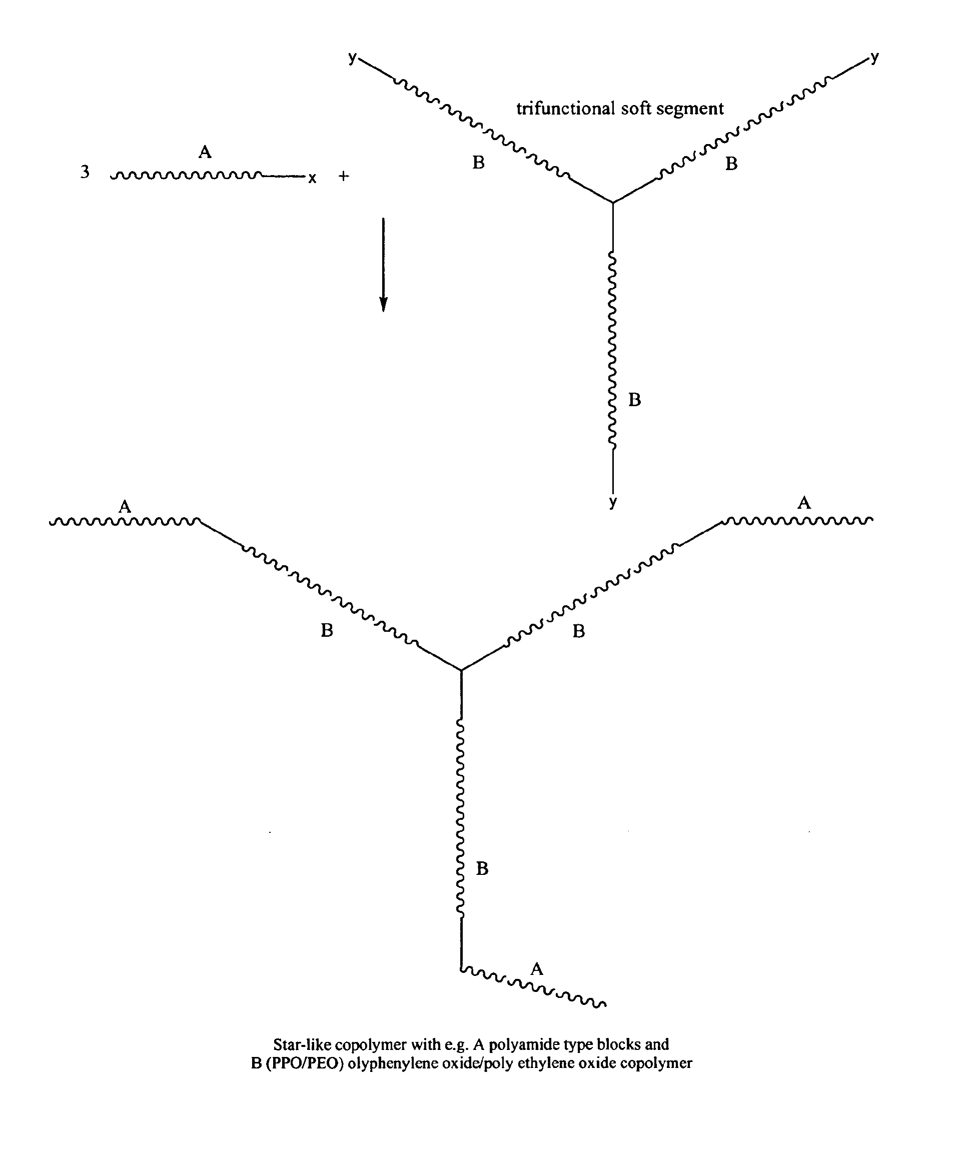

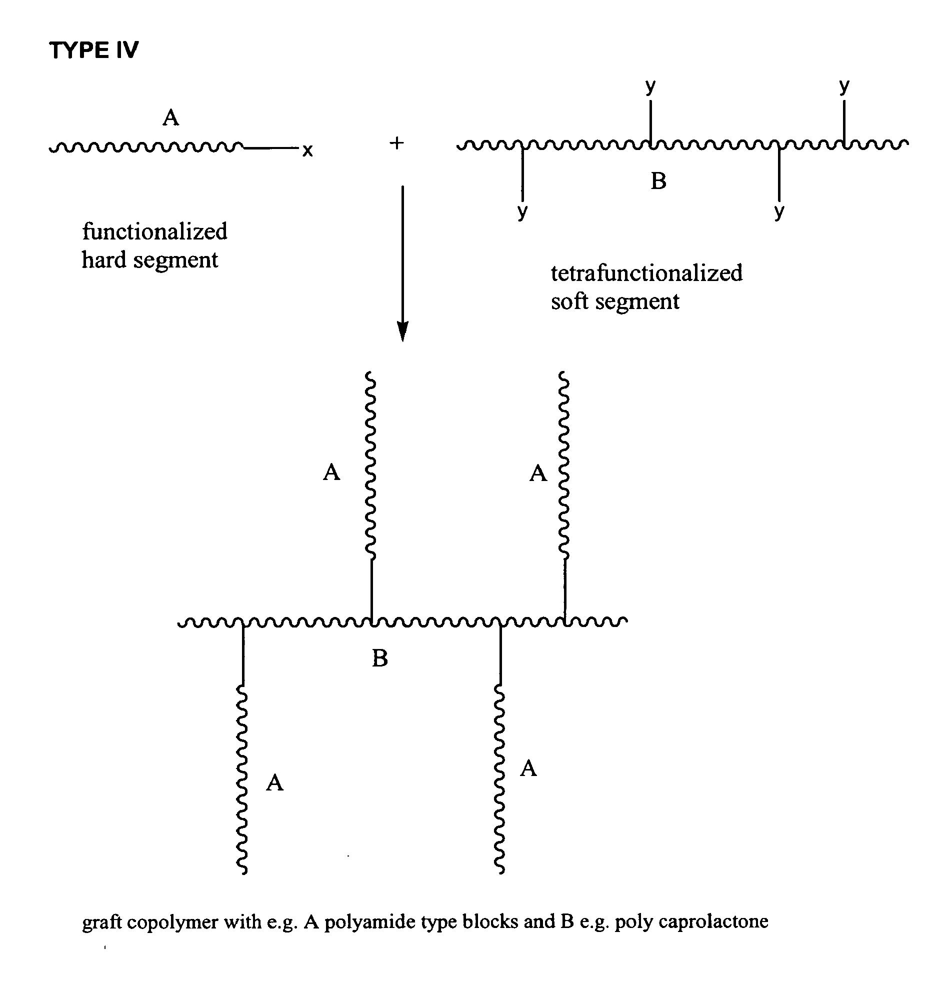

### EXPERIMENTAL EXAMPLES

### Example A: Prefunctionalizing a Polyamide (Oligomerization)

Following the instructions of Eldred et al. J. Am. Chem. Soc. 125 (2003), 3423 a polyamid (PA), Grilamid^{©} L25, a Nylon 12, is reacted with 1-Octadecylamin under addition of energy in form of heat and in presence of the catalyst Tris-(dimethylamino)-aluminium) in a stoichiometry of 1 mol PA added to 3 mol Amine. This results in a reduction of the original molar mass to 25% (8.550 g/mol). The overall reaction is shown below with 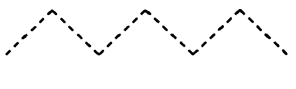 signifying the polyamid part.

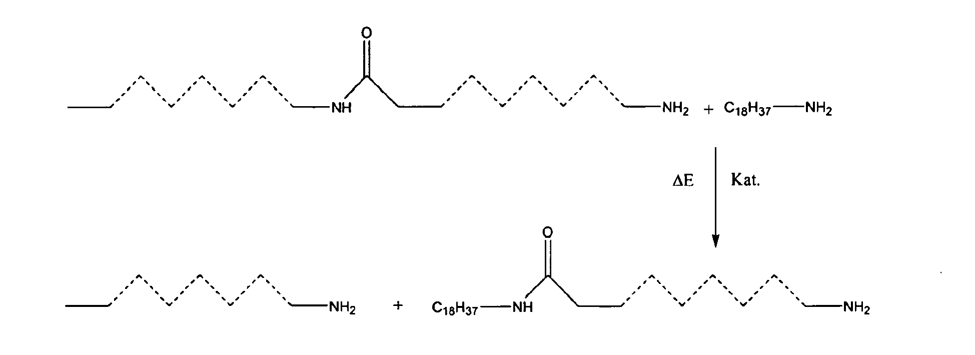

### Example B: Functionalizing a Polyamide (Oligo-PA-OX)

The Prefunctionalized Polyamide from Example A is reacted under addition of energy (heating) with 1,3-Phenylbisoxazoline in a stoichiometry of 1 mol prefunctionalized PA added to 1.1 mol Bisoxazoline. The overall reaction is shown below with 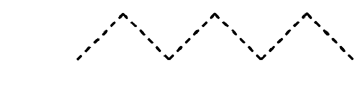 signifying the polyamid part.

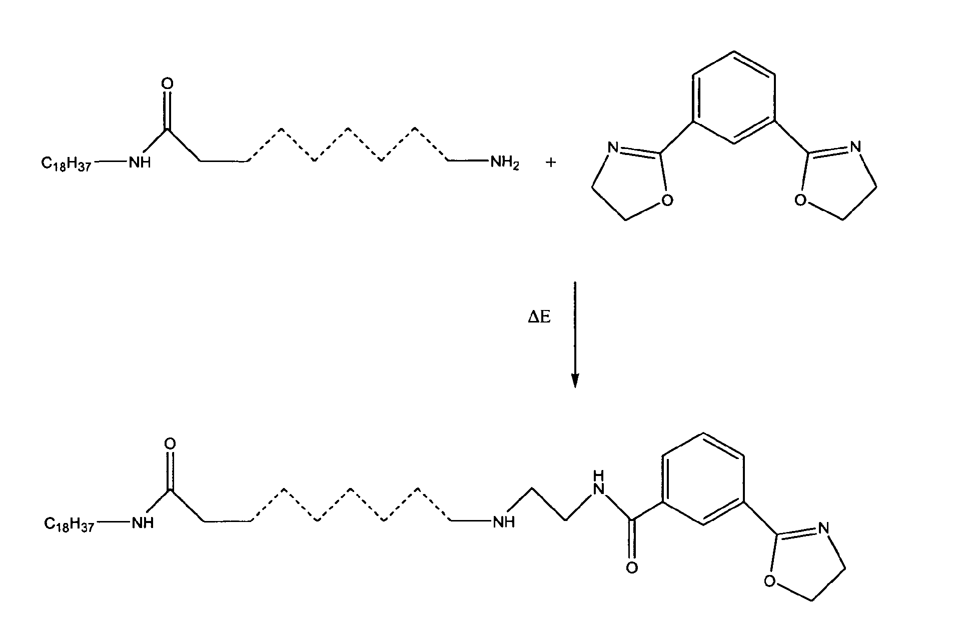

### Example 1A: Dendritic Polymer A (fast extrusion, no catalyst, 10% BOLTORN)

90% (weight) of Example B (Oligo-PA-Ox) is reacted with 10 % (weight) of BOLTORN® H40. This results in a stoichiometry of 7.7 mol Oligo-PA-Ox to 1 mol BOLTORN® H40. BOLTORN® H40 is a dendritic/highly branched Polyester structure with a calculated Mw of 7.316 g/mol and theoretically 64 free/primary OH groups per molecule. BOLTORN® can be acquired through Perstorp AB (Sweden). The reaction was carried out using reactive extrusion. No catalyst was added and the reactive extrusion was carried out with high speed.

### BOLTRON H40 is shown below:

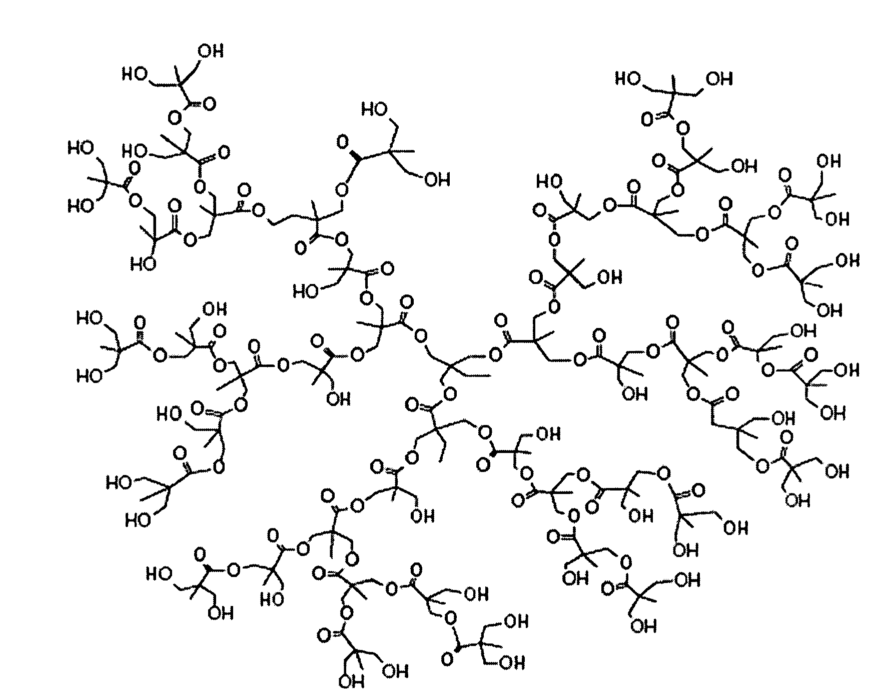

### Example 1B: Dendritic Polymer B (normal extrusion, no catalyst, 10% BOLTORN)

90% (weight) of Example B (Oligo-PA-Ox) is reacted with 10 % (weight) of BOLTORN^{®} H40. This results in a stoichiometry of 7.7 mol Oligo-PA-Ox to 1 mol BOLTORN® H40. The reaction was carried out using reactive extrusion. No catalyst was added and the reactive extrusion was carried out with normal speed.

### Example 1C: Dendritic Polymer C (normal extrusion, catalyst, 10% BOLTORN)

90% (weight) of Example B (Oligo-PA-Ox) is reacted with 10 % (weight) of BOLTORN^{®} H40. This results in a stoichiometry of 7.7 mol Oligo-PA-Ox to 1 mol BOLTORN® H40. The reaction was carried out using reactive extrusion. A transamidation catalyst (XXX, which ???) was added and the reactive extrusion was carried out with normal speed.

### Example 1D: Dendritic Polymer D (normal extrusion, catalyst, 15% BOLTORN)

85% (weight) of Example B (Oligo-PA-Ox) is reacted with 15 % (weight) of BOLTORN^{®} H40. This results in a stoichiometry of 4.9 mol Oligo-PA-Ox to 1 mol BOLTORN^{®} H40. The reaction was carried out using reactive extrusion. The transamidation catalyst (catalyst Tris-(dimethylamino)-aluminium) was added and the reactive extrusion was carried out with normal speed.

### Example 2A: Branched Block Co-Polymer A (3.74 % Polyol 3165)

96.26 % (weight) of Example B (Oligo-PA-Ox) is reacted with 3.74% (weight) of Polyol 3165. This results in a stoichiometry of 3 mol Oligo-PA-Ox to 1 mol Polyol 3165. Polyol 3165 is a functionalized polyethyleneoxide-polypropyleneoxide-copolymer (trifunctional OH-terminated PEO-PPO-Copolymer) Mw = 1.000 g/mol. Polyol can be acquired through Perstorp AB (Sweden). The reaction was carried out using reactive extrusion.

### Polyol 3165 is shown below:

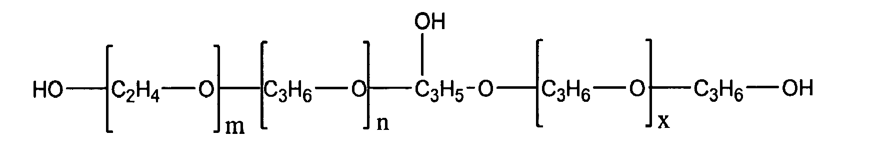

### Example 2B: Branched Block Co-Polymer B (3.00 % Polyol 3165 + 19.8 % Nylon 12)

77.2 % (weight) of Example B (Oligo-PA-Ox) is reacted with 3.74% (weight) of Polyol 3165 and 19.8 % (weight) of Grilamid L25. Grilamid L25 is a heat and UV stabilized Nylon 12 to be purchased through EMS-Grivory. This results in a stoichiometry of 3 mol Oligo-PA-Ox to 1 mol Polyol 3165. The reaction was carried out using reactive extrusion.

### Example 2C: Branched Block Co-Polymer C (5.50 % Polyol 3165)

94.50 % (weight) of Example B (Oligo-PA-Ox) is reacted with 5.50% (weight) of Polyol 3165. This results in a stoichiometry of 2 mol Oligo-PA-Ox to 1 mol Polyol 3165. The reaction was carried out using reactive extrusion.

### Test of mechanical properties:

### Test 1

The material according to examples 1 A, 1B, 1C, 1D, 2A, 2B, or 2C was tested together with comparative samples of PEBAX and Nylon 12 (Grilamid L25). Inall cases n was 5 and the speed v was 100 mm/min. as can be seen the new materials were in (nearly) all aspects, especially the most prefered examples 1 A and 2A in all aspects in the middle between Nylon 12 and PEBAX as was the intention of this invention.

**Table I:**

| **Example:** | **Nylon 12** | **Pebax** | **Ex.1A** | **Ex. 2A** | **Ex. 1B** | **Ex. 2B** | **Ex. 2C** | **Ex. 1C** | **Ex. 1D** |
|---|---|---|---|---|---|---|---|---|---|
| **Tensile Strength [MPa]** | 51.1 | 39.5 | 48.5 | 47.6 | 45.3 | 50.1 | 45.7 | 43.0 | 41.7 |
| **Tensile strain at tensile strength [%]** | 306 | 475 | 379 | 419 | 350 | 351 | 340 | 254 | 122 |
| **Yield Stress [MPa]** | 44.7 ± 0.25 | 26.5 ± 0.22 | 38.7 ± 0.36 | 37.6 ±.26 | 38.1 | 39.2 | 36.0 | 37.3 | 39.1 |
| **Yield Strain [%]** | 4.6 ±0.1 | 17.8 ±0.83 | 10.7 ±0.67 | 10.9 ±0.81 | 11.3 | 10.8 | 10.9 | 11.7 | 8.9 |
| **Tensile stress at break [MPa]** | 47.0 ± 0.15 | 31.5 ± 1.36 | 45.0 ± 1.96 | 44.2 ± 1.17 | 43.4 | 47.1 | 43.8 | 40.9 | 35.1 |
| **Tensile strain at break [%]** | 313 ± 23 | 491 ± 2 | 390 ± 42,1 | 429 ± 5.8 | 355 | 357 | 347 | 298 | 260 |
| **Secant modulus** | 1362 | 502 | 866 | 979 | 816 | 1050 | 959 | 781 | 894 |
| **Tensile-E-Modulus [Mpa]** | 1407 ± 66 | 517 ± 7 | 883 ± 19.3 | 994 ± 11.5 | 831 | 1065 | 973 | 789 | 904 |

### Test 2

In another test for mechanical properties the material according to examples 1A, and 2A was tested together with comparative samples of PEBAX and Nylon 12 (Grilamid L25). The results are shown in Figures 1, 2 and 3.
As can be seen in Figure 1) the E-module in radial and longitudinal direction was for both examples in the middle between Nylon 12 and PEBAX.

As can be seen in Figure 2) the compliance curve was for both examples flat and close to Nylon 12, showing the preferred more linear behaviour.
As can be seen in Figure 3) longitudinal growth is for both examples as intended closer to Nylon and between Nylon 12 and PEBAX.

## Claims

1. Medical device comprising a non-linear block-co-polymer.

2. Medical device according to claim 1, wherein the medical device is selected from implanted or implantable medical devices, preferably balloon/balloon material, stents, stent grafts, grafts, graft connectors or catheters, most preferably is a balloon/balloon material.

3. Medical device according to claim 1, wherein the medical device is a balloon attached to a balloon catheter.

4. Medical device according to claim 3, wherein the balloon is consisting of the non-linear block-co-polymer.

5. Medical device according to any of claims 1 to 4, wherein the non-linear block-co-polymer is selected from branched polyamides, a branched or grafted Block-Co-Polymer or a dendritic system carrying polyamides.

6. Non-linear block-co-polymer comprising at least four polymer segments of which at least one is a hard segment and at least one is a soft segment.

7. Non-linear block-co-polymer according to claim 6 being selected from a branched polyamide, a branched or grafted block-co-polymer or a dendritic system carrying polyamides.

8. Non-linear block-co-polymer according to any of claims 6 or 7, wherein the block-co-polymer comprises at least three hard segments covalently bound directly or through a linker to at least one soft segment
or
at least three soft segments covalently bound directly or through a linker to at least one hard segment.

9. Non-linear block-co-polymer according to claim 6, wherein the non-linear block-co-polymer has a structure selected from one of the following general formulas: Type II A or B, Type III A or B, Type IV A or B, Type V A or B, Type VI A or B, Type VII A or B, Type VIII A or B; Type IX A or B, or Type X: 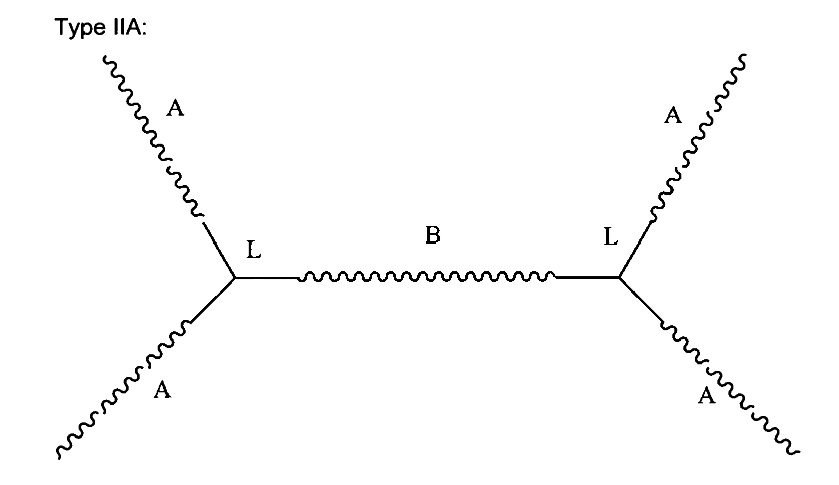 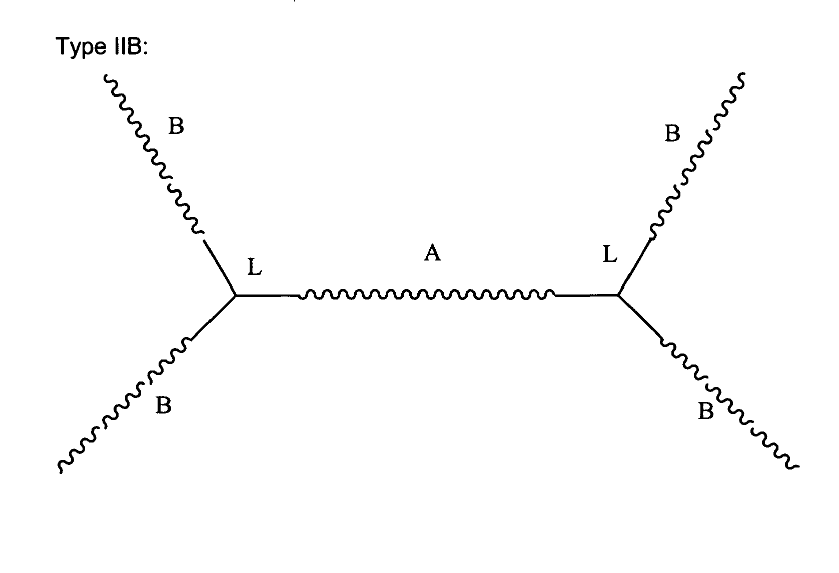 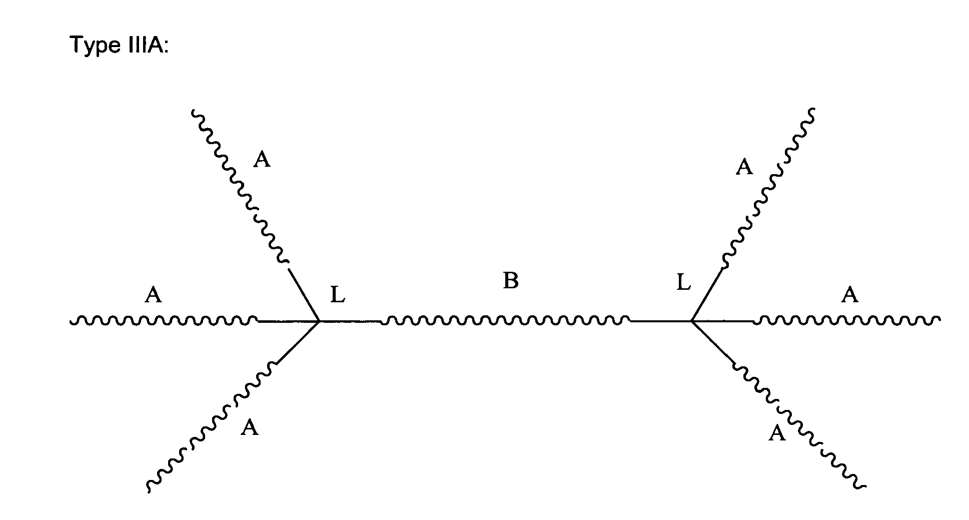 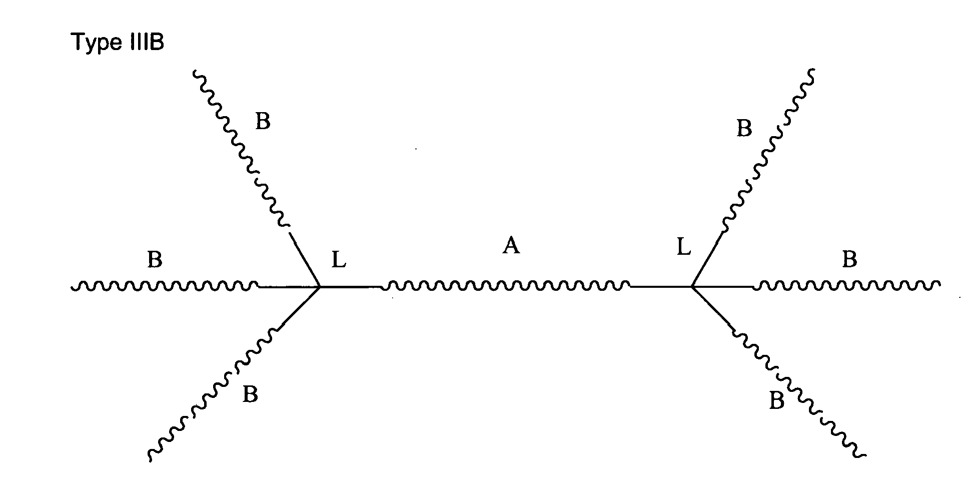 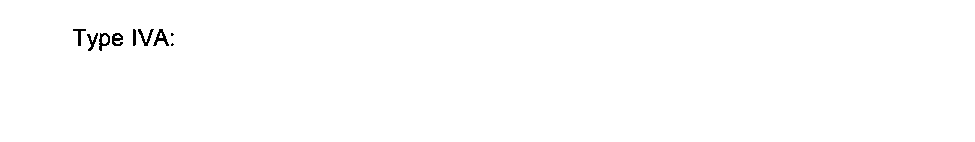 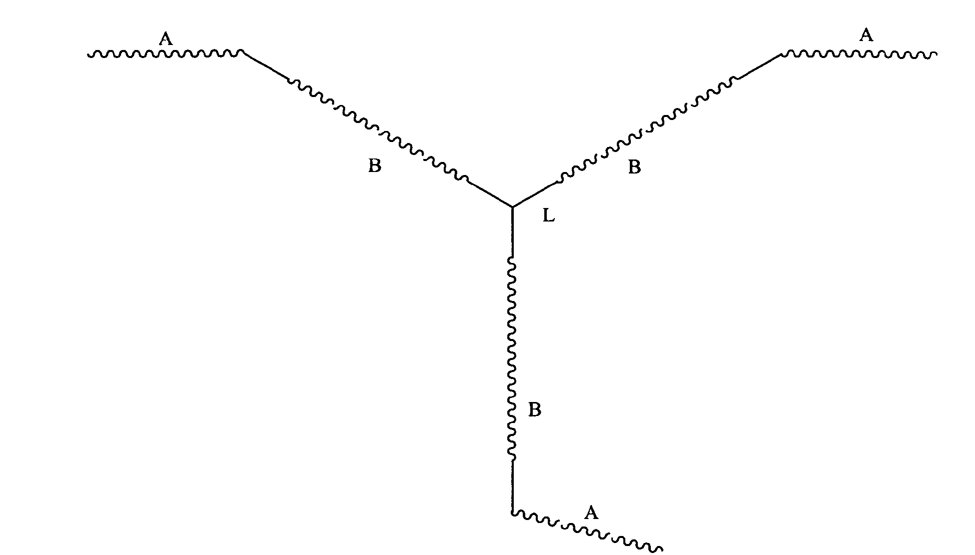 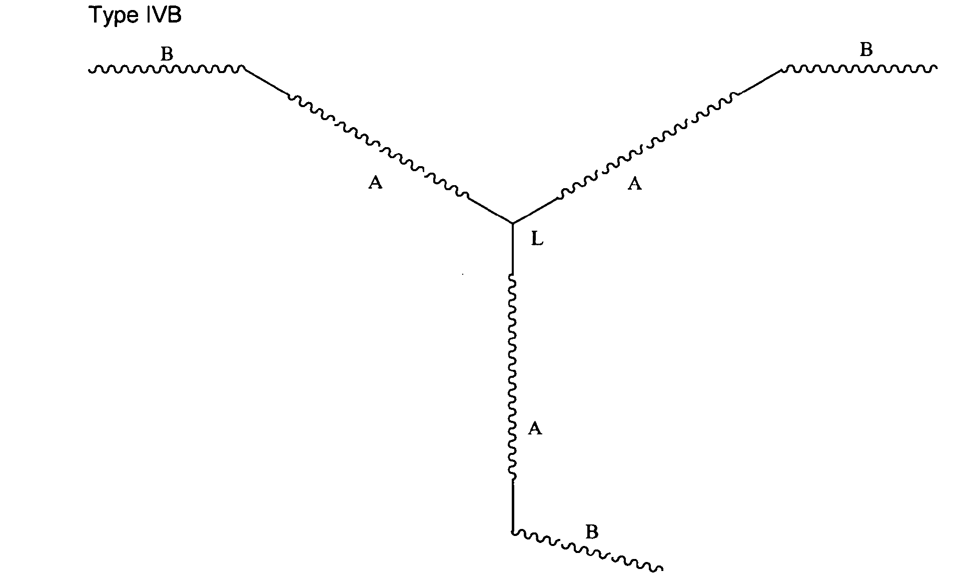 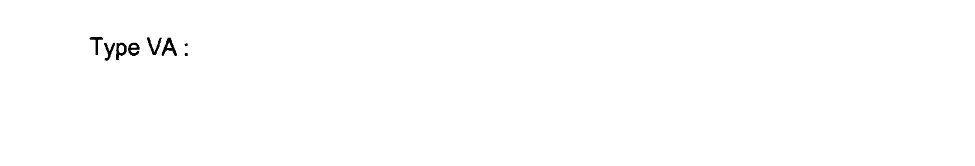 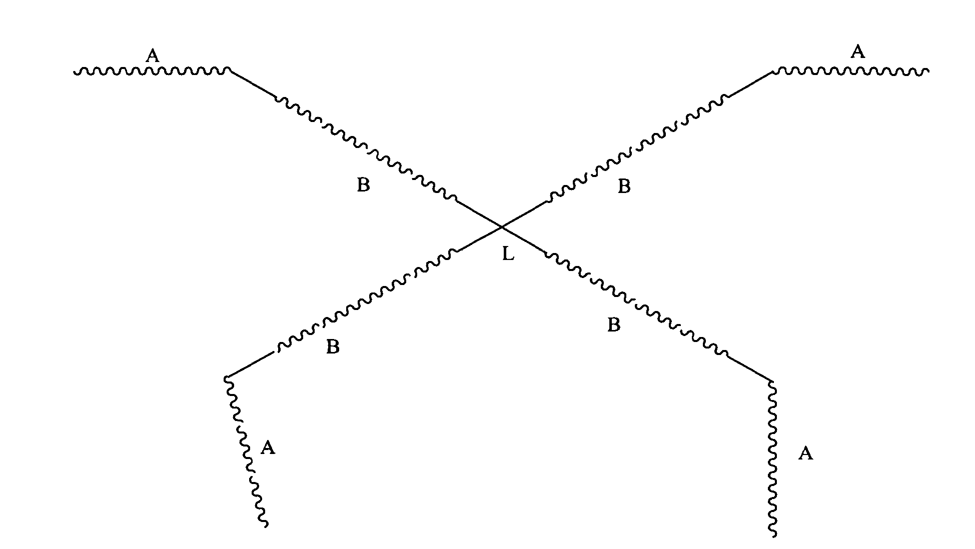 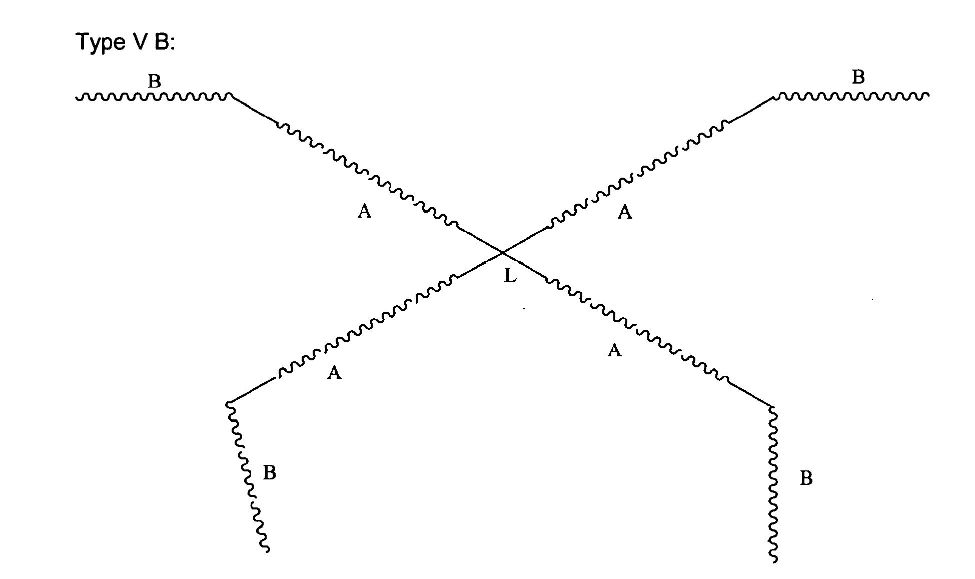 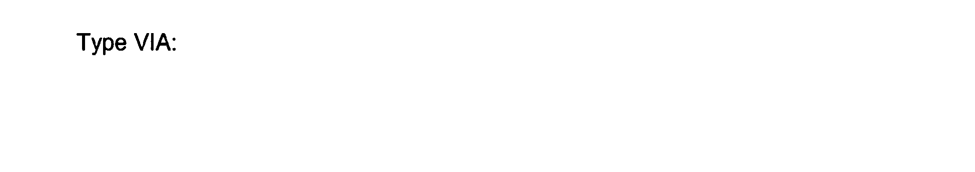 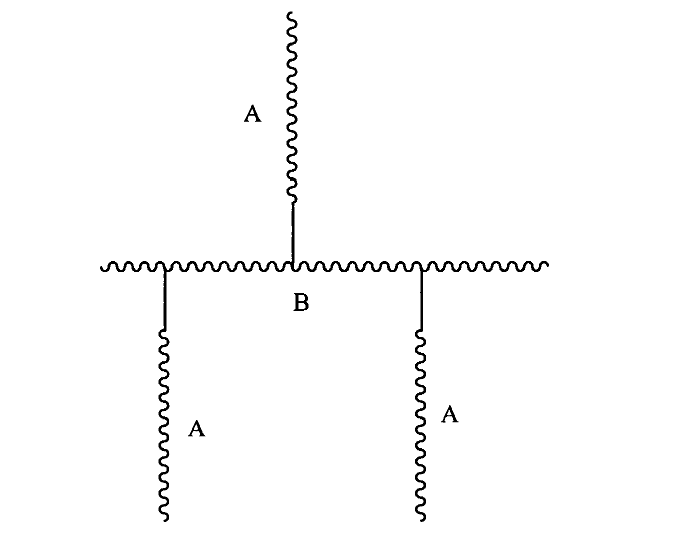 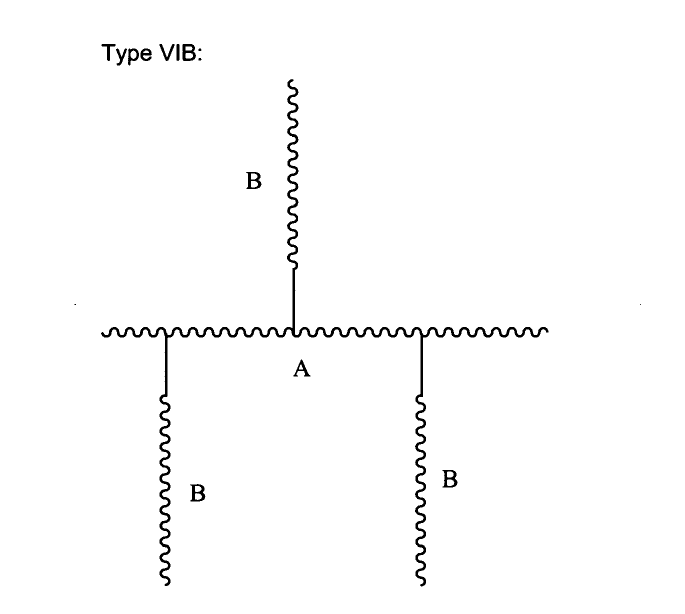 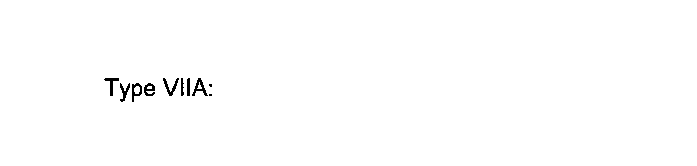 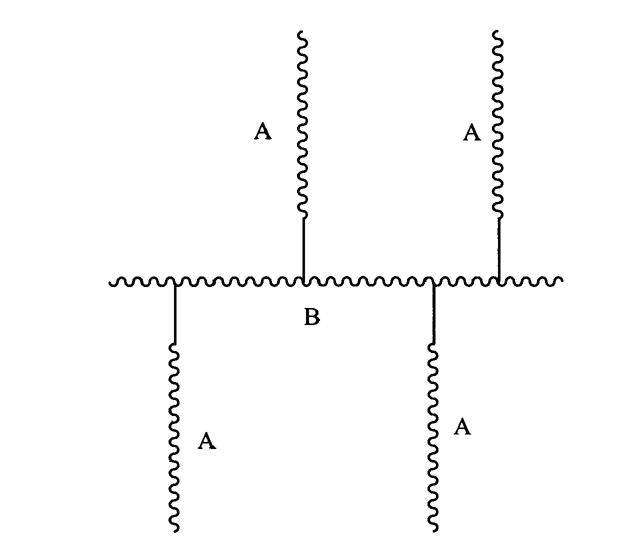 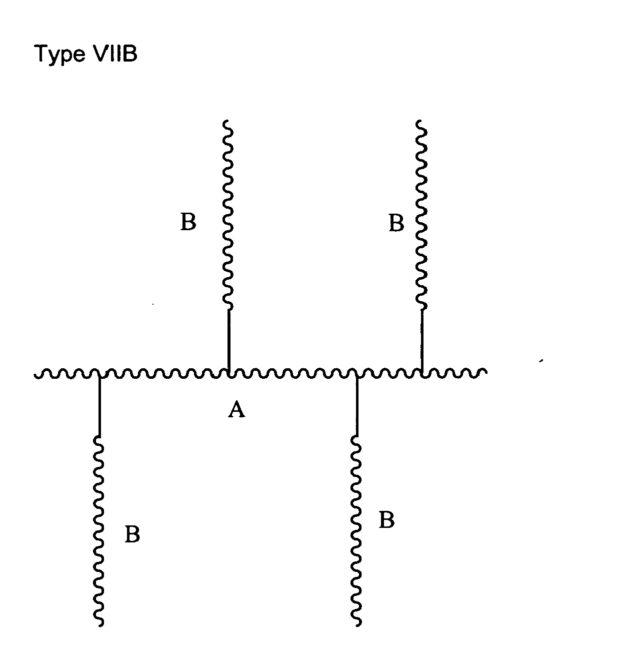 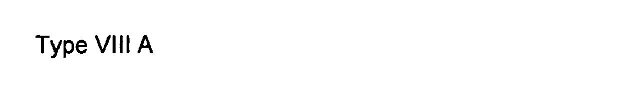 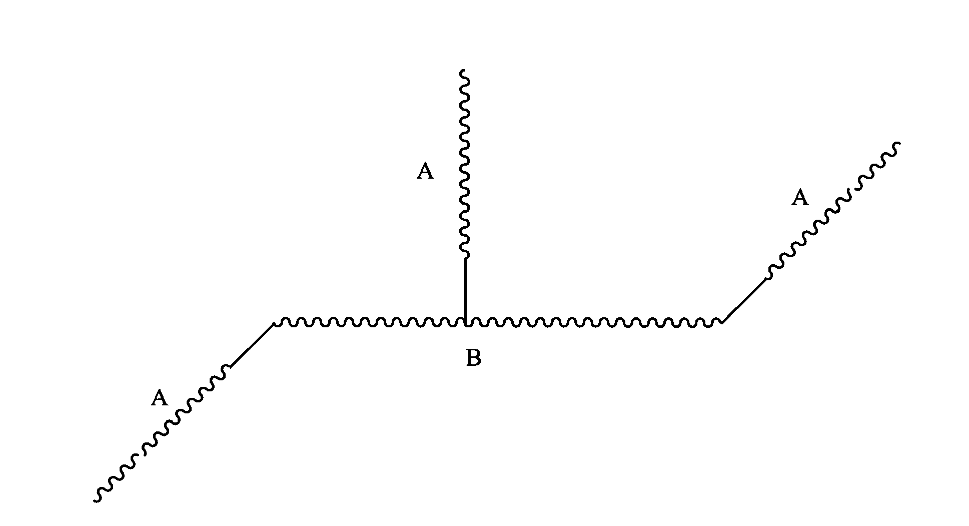 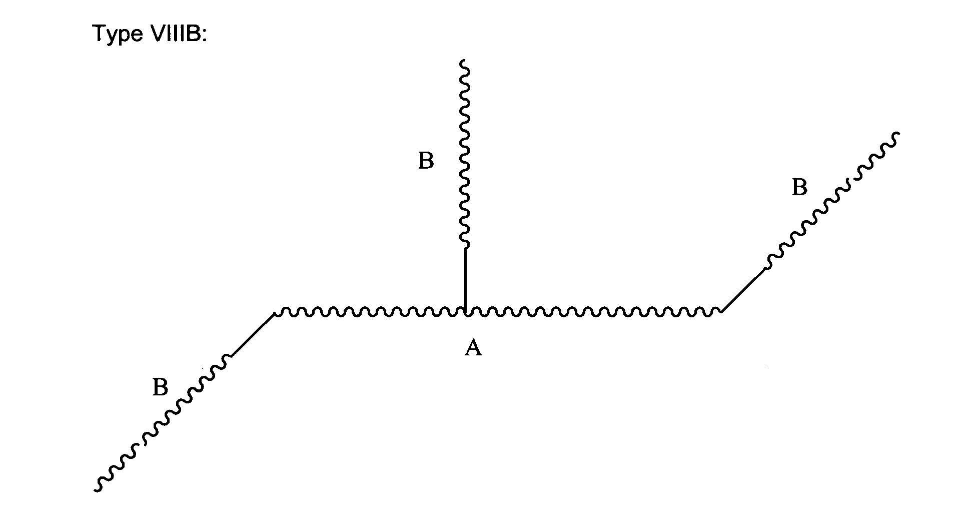 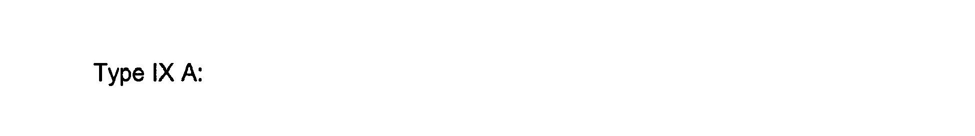 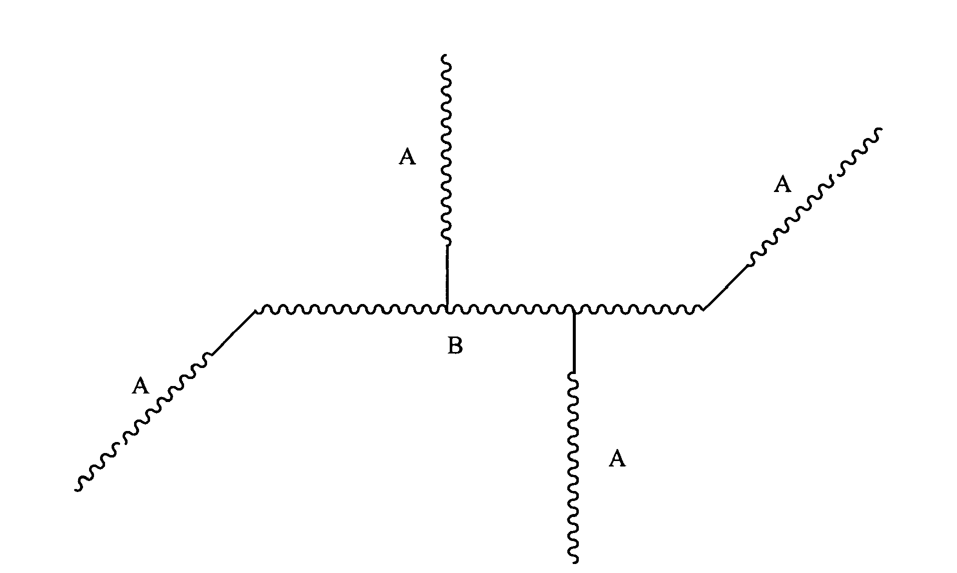 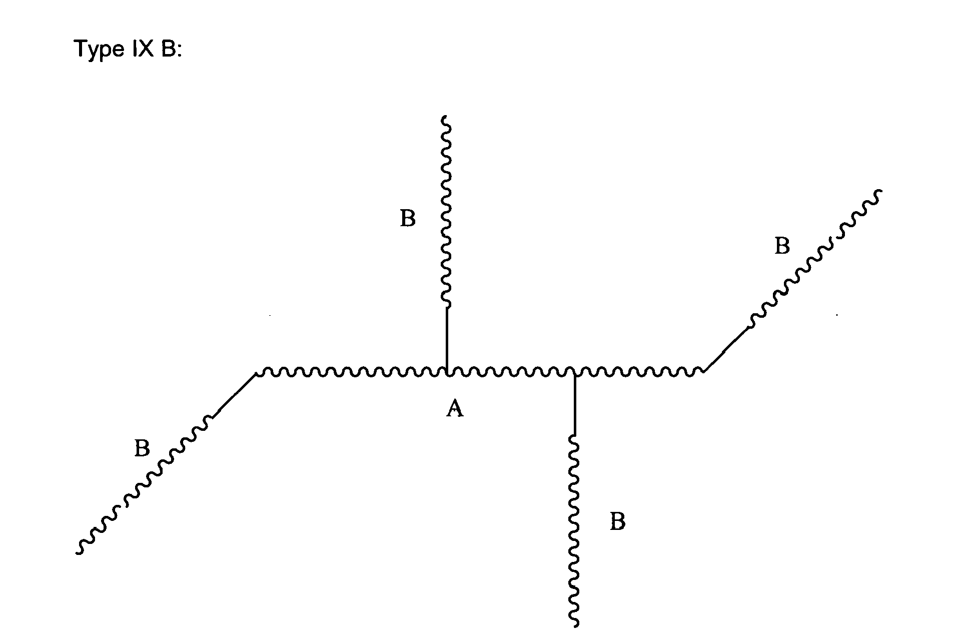 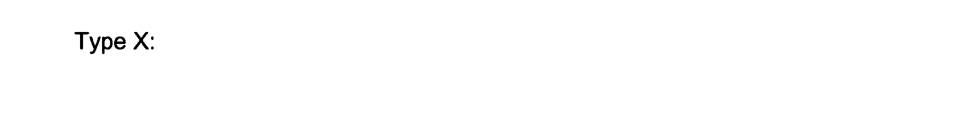 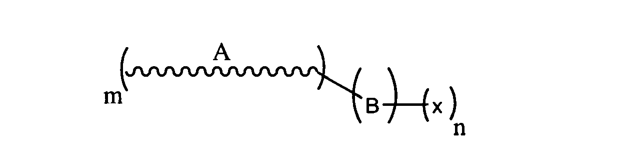 , wherein
A is a hard segment,
B is a soft segment;
x is a functional group;
m is a number between 3 and 15,
n is a number between 0 and 60;
and n+ m is a number between 3 and 70;
_ is an optional coupling reagent; and
L is a linker.

10. Non-linear block-co-polymer according to any of claims 6 to 9, wherein the block-co-polymer comprises at least three hard segments covalently bound directly or through a coupling reagent to at least one soft segment.

11. Non-linear block-co-polymer according to claim 10, wherein the block co-polymer has a structure selected from one of the following general formulas: Type II A, Type III A, Type IV A, Type V A, Type VI A, Type VII A, Type VIII A, Type IX A and Type X: 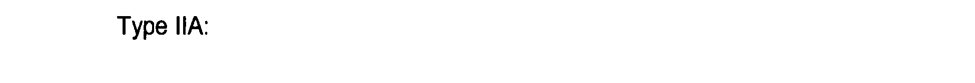 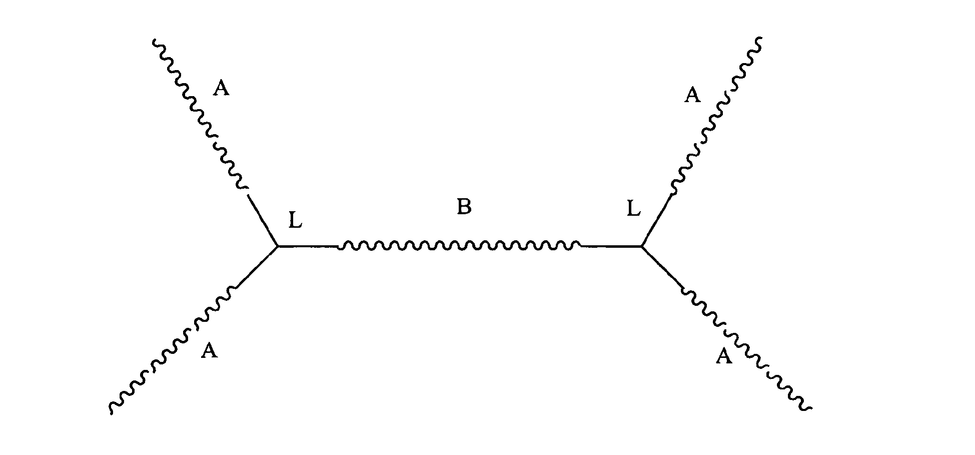 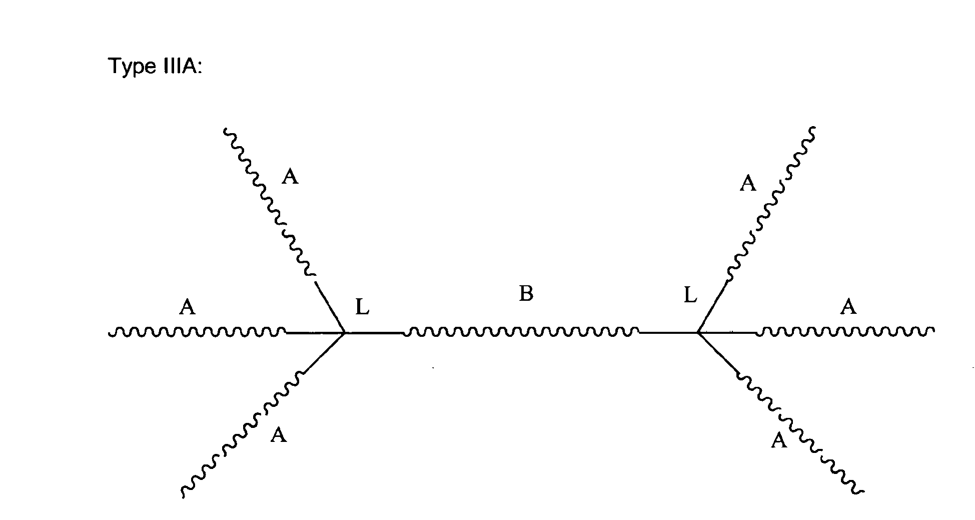 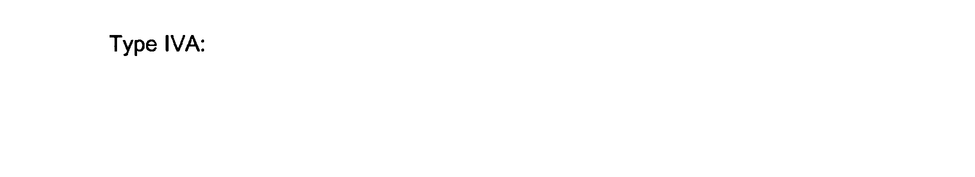 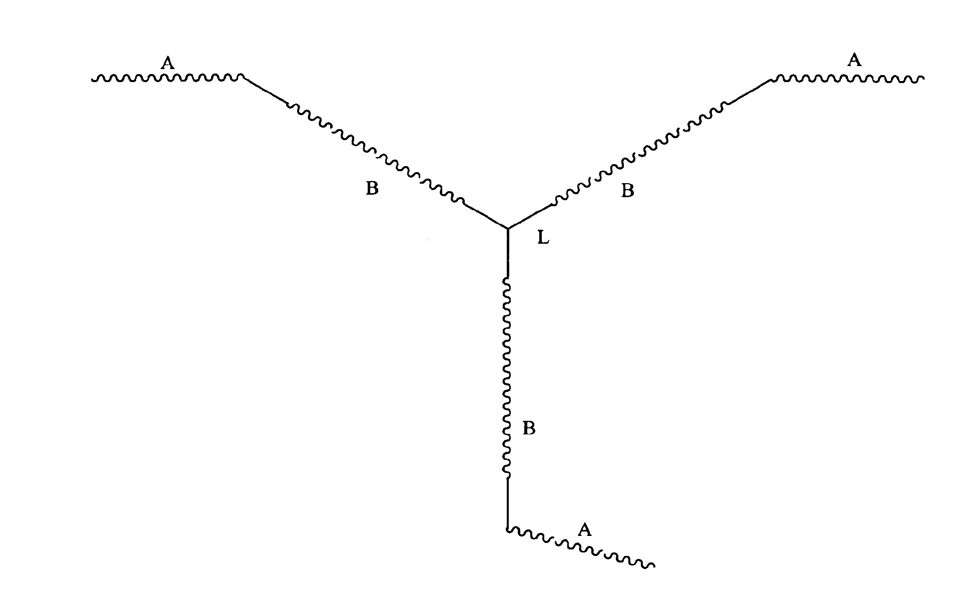 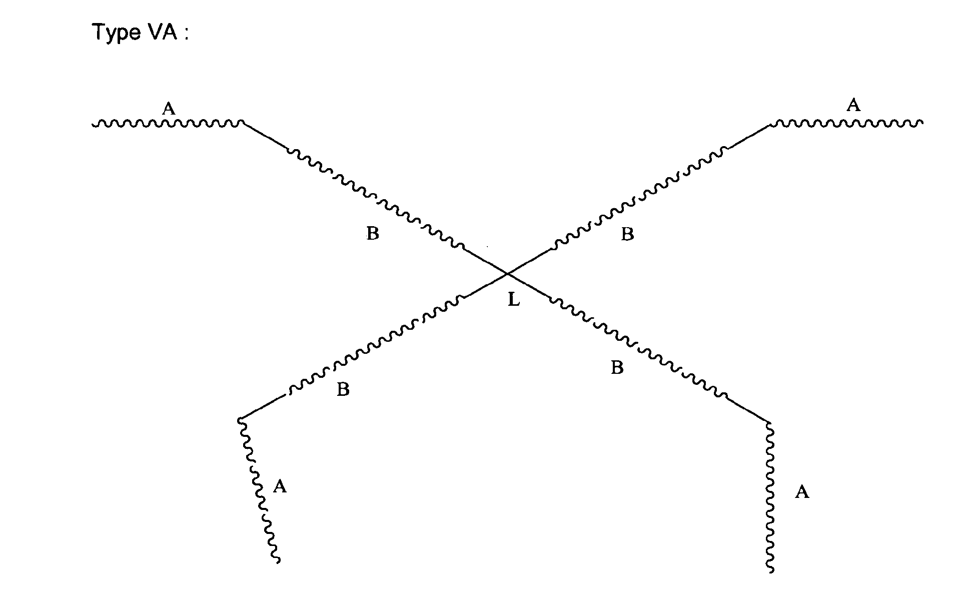 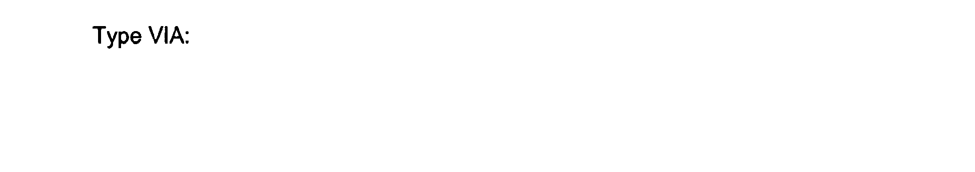 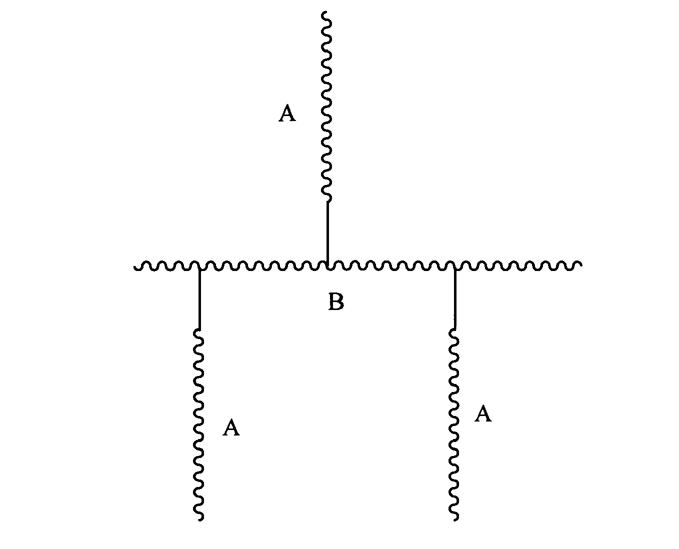 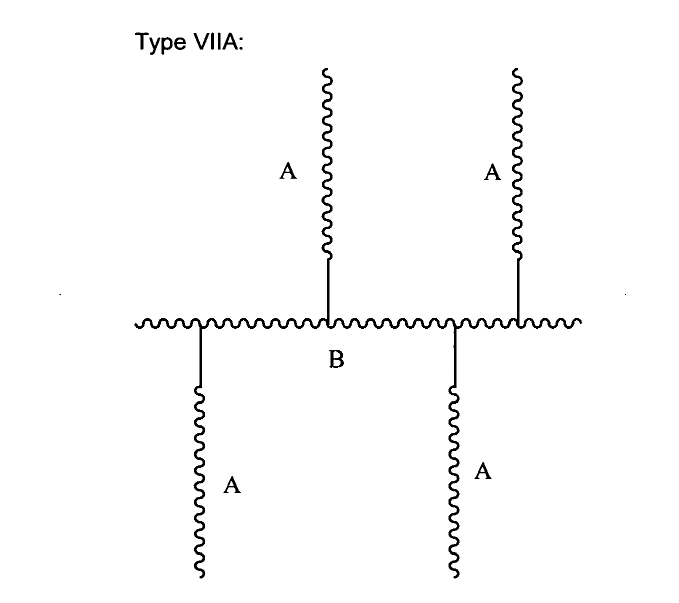 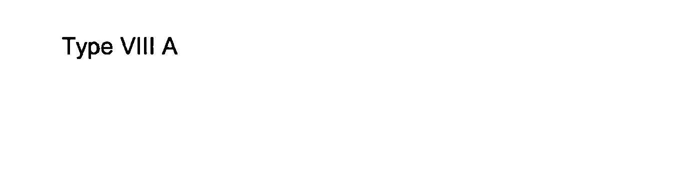 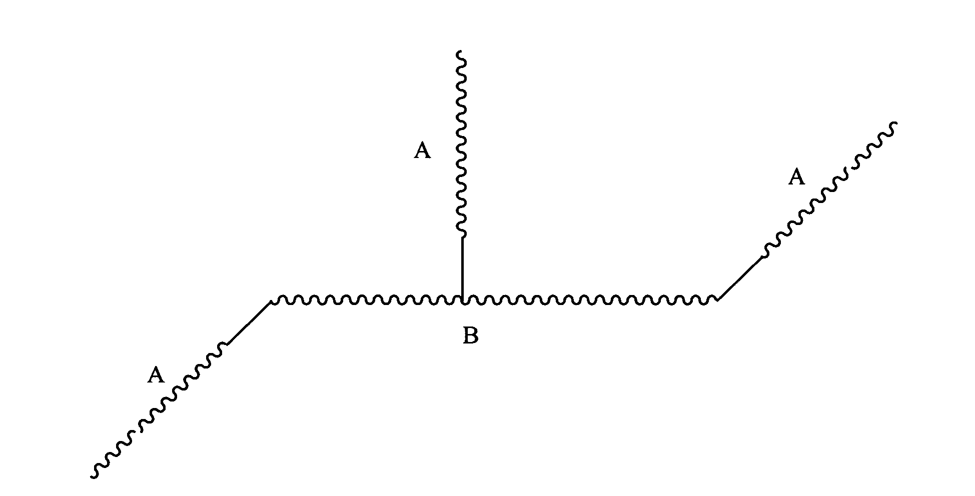 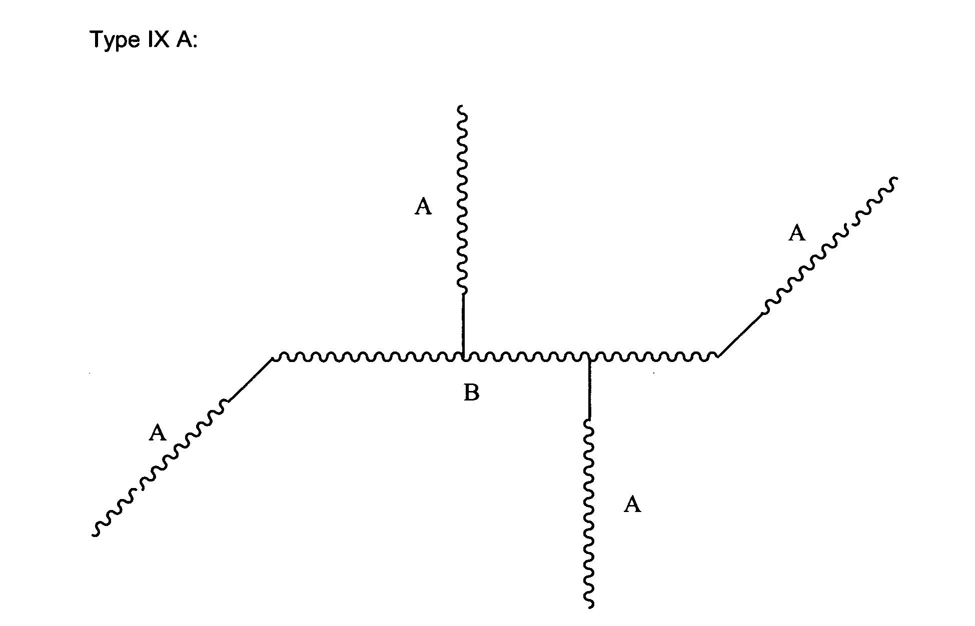 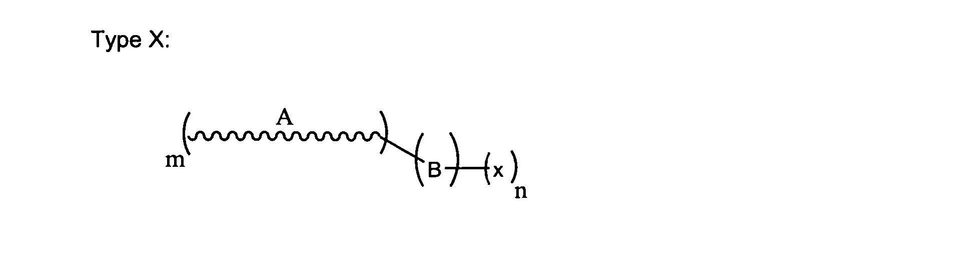 , wherein
A is a hard segment,
B is a soft segment;
x is a functional group;
m is a number between 3 and 15,
n is a number between 0 and 60;
and n+ m is a number between 3 and 70;
_ is an optional coupling reagent; and
L is a linker.

12. Non-linear block-co-polymer according to any of claims 6 to 11, wherein the hard segments are functionalized; preferably are functionalized showing at least one reactive group selected from epoxide, COOH, NH₂, or OH; more preferably are mono-functionalized, most preferably are mono-functionalized showing at least one reactive group selected from epoxide, COOH, NH₂, or OH.

13. Non-linear block-co-polymer according to any of claims 6 to 12, wherein the hard segments are polyamides, preferably are functionalized polyamides; preferably are functionalized polyamides showing at least one reactive group selected from epoxide, COOH, NH₂, or OH; most preferably are mono-functionalized polyamides showing at least one reactive group selected from epoxide, COOH, NH₂, or OH.

14. Non-linear block-co-polymer according to claim 13, wherein the polyamides are functionalized by a reagent selected from
• aliphatic diamines like octadecyldiamine;
• 2-piperazinoethylamine; or
• trimellitic anhydride.

15. Non-linear block-co-polymer according to any of claims 13 to 14, wherein the functionalized polyamides are low-molecular polyamides.

16. Non-linear block-co-polymer according to claim 6 to 16, wherein at least one of the hard segments is covalently bound through a coupling reagent to at least one soft segment, wherein the coupling reagent is preferably selected from
• biphenyl tetracarboxylic dianhydride;
• tris(2-aminoethyl)amine;
• trimethylpropane trisaminopropylene glycol ether;
• glycerol-propoxylate-triglycidylether;
• carbonyl biscaprolactam; or
• 1,3-phenylbisoxazolin; 1,3-phenylbisoxazolin.

17. Non-linear block-co-polymer according to any of claims 6 to 16, wherein the soft segment/s is/are functionalized soft segment/s; preferably is/are functionalized soft segments selected from
• Polyethers;
• Polyesters;
• Polydimethylsiloxanes; or
• Siloxylated polyether dioles
preferably selected from
• Polyether;
• Polyethylenoxid-Polypropyleneoxid-Copolymer;
• Polytetramethyleneoxyde (Polytetrahydrofurane);
• Polyester;
• Tetra-OH-functionalized polyester;
• Dendritic Polyester;
• Polycaprolactone;
• Polydimethylsiloxane; or
• Siloxylated polyether diole,
most preferably selected from
• Polyethylenoxid-Polypropyleneoxid-Copolymer; or
• Dendritic Polyester Boltorn H40.

18. Non-linear block-co-polymer according to any of claims 6 to 14, wherein the hard segments are polyamides functionalized by 1-Octadecylamine and preferably coupled to 1,3-Phenylbisoxazoline.

19. Non-linear block-co-polymer according to any of claims 6 to 14, wherein the soft segment segement is selected from Polyethylenoxid-Polypropyleneoxid-Copolymer; or Boltorn H40 and the hard segments are a polyamide functionalized by 1-Octadecylamine and coupled to 1,3-Phenylbisoxazoline.

20. Medical device according to any claims 1 to 5, wherein the non-linear block-co-polymer is a polymer according to any of claims 6 to 19.

21. Block-co-polymer, wherein the polymer is of general formula Type IA or Type IB 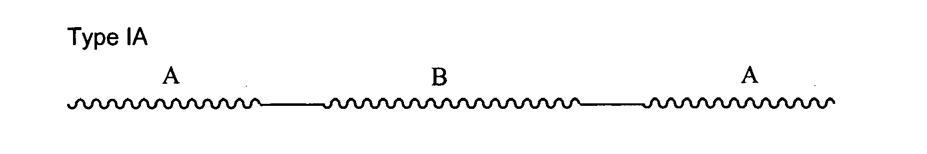 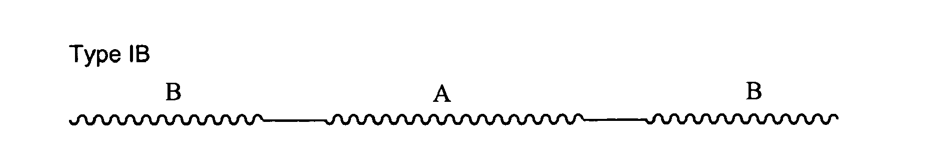 , wherein
A is a hard segment; preferably a functionalized polyamide;
_ is an optional coupling reagent; and
B is a soft segment, preferably a functionalized polyamide.

22. Medical device comprising a block-co-polymer according to claim 21.

23. Medical device according to claim 22, wherein the medical device is selected from implanted or implantable medical devices, preferably balloon/balloon material, stents, stent grafts, grafts, graft connectors, filters, embolic protection devices, closure devices, delivery systems, catheters and medical tubings, more preferably from balloons, balloon materials, catheters or medical tubings.

24. Use of a block-co-polymer, preferably a non-linear block-co-polymer, selected from branched polyamides, a branched or grafted Block-Co-Polymer or a dendritic system carrying polyamides in the production of implants or medical devices.

25. Use according to claim 24, wherein the implants or medical devices are implanted or implantable medical devices, preferably are balloons/balloon material, stents, stent grafts, grafts graft connectors, filters, embolic protection devices, closure devices, delivery systems, catheters and medical tubings, most preferably are balloons, balloon materials, catheters or medical tubings.

26. Use according to any of claims 24 to 25, wherein the block-co-polymer is a non-linear block-co-polymer according to any one of claims 6 to 19 or is a block-co-polymer according to claim 20.
